# EUROPEAN PATENT APPLICATION

(11) **EP 1 127 894 A1**
(43) Date of publication of application: **29.08.2001**
(21) Application number: 01108876.2
(22) Date of filing: 29.05.1998
(51) Int. Cl.: C07K 14/705, C07K 14/78, C07K 16/28, A61K 38/39

(54) **Method of diagnosis of transmissible spongiform encephalopathy (TSE)**

(30) Priority: 30.05.1997 EP 97108712
(62) Divisional of application: 98929404.6
(71) Applicant: Weiss, Stefan, 80796 München (DE)
(72) Inventor: Weiss, Stefan, 80796 München (DE)
(74) Representative: Henkel, Feiler, Hänzel

(57) **Abstract**

The present invention relates to a method of diagnosis of transmissible spongiform encephalopathy (TSE), said method comprising the following step of determining the overexpression of a marker protein for TSE in a sample to be tested. The present invention also relates to a method of diagnosis of transmissible spongiform encephalopathy (TSE), said method comprising the steps of:
(a) determining the expression of a marker protein for TSE in a sample to be tested; and
(b) comparing the value obtained with a control value, wherein an increase in the expression level is indicative of the occurence of TSE. The present invention also relates -to a diagnostic test kit for detecting a transmissible spongiform encephalopathy (TSE), characterized in that it contains antibodies specific to the laminin receptor precursor (LRP) and/or the laminin receptor (LR) for detecting the altered expression of the laminin receptor (LR) and/or the laminin receptor precursor (LRP) as a marker protein for TSE by a method mentioned above. In addition the present invention relates to the use of an antibody capable of binding the laminin receptor (LR) and/or the laminin receptor precursor (LRP) for the preparation of a composition for diagnosing a transmissible spongiform encephalopathy (TSE).

SEQUENCE LISTING

(1) GENERAL INFORMATION:

(i) APPLICANT:
(A) NAME: WINNACKER, Ernst-Ludwig
(B) STREET: Dall'Armistrasse 41a
(C) CITY: Muenchen
(E) COUNTRY: DE
(F) POSTAL CODE (ZIP): 80638

(A) NAME: WEISS, Stefan
(B) STREET: Bluetenstrasse 20
(C) CITY: Muenchen
(E) COUNTRY: DE
(F) POSTAL CODE (ZIP): 80799

(A) NAME: EDENHOFER, Frank
(B) STREET: Bernd-Alois-Zimmermann-Strasse 2
(C) CITY: Erfstadt
(E) COUNTRY: DE
(F) POSTAL CODE (ZIP): 50374

(A) NAME: RIEGER, Roman
(B) STREET: Roemerstrasse 43
(C) CITY: Weilheim
(E) COUNTRY: DE
(F) POSTAL CODE (ZIP): 82362

(ii) TITLE OF INVENTION: Pharmaceutical compositions comprising a
soluble laminin receptor precursor or a compound which
blocks the interaction of the laminin receptor precursor
and PrPSc or PrPc

(iii) NUMBER OF SEQUENCES: 3

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: PCT/EP98/03220
(vi) PRIOR APPLICATION DATA:
(A) APPLICATION NUMBER: EP 97 10 8712.7
(B) FILING DATE: 30-MAY-1997

(2) INFORMATION FOR SEQ ID NO: 1:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO: 1:

CTATGCTACT AGTAAATCAG TCAC 24

(2) INFORMATION FOR SEQ ID NO: 2:

(i) SEQUENCE CHARACTERISTICS:
(A) LENGTH: 59 base pairs
(B) TYPE: nucleic acid
(C) STRANDEDNESS: single
(D) TOPOLOGY: linear

(xi) SEQUENCE DESCRIPTION: SEQ ID NO: 2:

CCCATGGCCC GGGATCCCTT GTCATCGTCG TCCTTGTAGT CCGCAAAGGC AGAATGCGC 59

(2) INFORMATION FOR SEQ ID NO: 3:

(i) SEQUENCE CHARACTERISTICS:
(A) LENGTH: 9780 base pairs
(B) TYPE: nucleic acid
(C) STRANDEDNESS: single
(D) TOPOLOGY: linear

(xi) SEQUENCE DESCRIPTION: SEQ ID NO: 3:

AAGCTTTACT CGTAAAGCGA GTTGAAGGAT CATATTTAGT TGCGTTTATG AGATAAGATT 60

GAAAGCACGT GTAAAATGTT TCCCGCGCGT TGGCACAACT ATTTACAATG CGGCCAAGTT 120

ATAAAAGATT CTAATCTGAT ATGTTTTAAA ACACCTTTGC GGCCCGAGTT GTTTGCGTAC 180

GTGACTAGCG AAGAAGATGT GTGGACCGCA GAACAGATAG TAAAACAAAA CCCTAGTATT 240

GGAGCAATAA TCGATTTAAC CAACACGTCT AAATATTATG ATGGTGTGCA TTTTTTGCGG 300

GCGGGCCTGT TATACAAAAA AATTCAAGTA CCTGGCCAGA CTTTGCCGCC TGAAAGCATA 360

GTTCAAGAAT TTATTGACAC GGTAAAAGAA TTTACAGAAA AGTGTCCCGG CATGTTGGTG 420

GGCGTGCACT GCACACACGG TATTAATCGC ACCGGTTACA TGGTGTGCAG ATATTTAATG 480

CACACCCTGG GTATTGCGCC GCAGGAAGCC ATAGATAGAT TCGAAAAAGC CAGAGGTCAC 540

AAAATTGAAA GACAAAATTA CGTTCAAGAT TTATTAATTT AATTAATATT ATTTGCATTC 600

TTTAACAAAT ACTTTATCCT ATTTTCAAAT TGTTGCGCTT CTTCCAGCGA ACCAAAACTA 660

TGCTTCGCTT GCTCCGTTTA GCTTGTAGCC GATCAGTGGC GTTGTTCCAA TCGACGGTAG 720

GATTAGGCCG GATATTCTCC ACCACAATGT TGGCAACGTT GATGTTACGT TTATGCTTTT 780

GGTTTTCCAC GTACGTCTTT TGGCCGGTAA TAGCCGTAAA CGTAGTGCCG TCGCGCGTCA 840

CGCACAACAC CGGATGTTTG CGCTTGTCCG CGGGGTATTG AACCGCGCGA TCCGACAAAT 900

CCACCACTTT GGCAACTAAA TCGGTGACCT GCGCGTCTTT TTTCTGCATT ATTTCGTCTT 960
TCTTTTGCAT GGTTTCCTGG AAGCCGGTGT ACATGCGGTT TAGATCAGTC ATGACGCGCG 1020
TGACCTGCAA ATCTTTGGCC TCGATCTGCT TGTCCTTGAT GGCAACGATG CGTTCAATAA 1080
ACTCTTGTTT TTTAACAAGT TCCTCGGTTT TTTGCGCCAC CACCGCTTGC AGCGCGTTTG 1140
TGTGCTCGGT GAATGTCGCA ATCAGCTTAG TCACCAACTG TTTGCTCTCC TCCTCCCGTT 1200
GTTTGATCGC GGGATCGTAC TTGCCGGTGC AGAGCACTTG AGGAATTACT TCTTCTAAAA 1260
GCCATTCTTG TAATTCTATG GCGTAAGGCA ATTTGGACTT CATAATCAGC TGAATCACGC 1320
CGGATTTAGT AATGAGCACT GTATGCGGCT GCAAATACAG CGGGTCGCCC CTTTTCACGA 1380
CGCTGTTAGA GGTAGGGCCC CCATTTTGGA TGGTCTGCTC AAATAACGAT TTGTATTTAT 1440
TGTCTACATG AACACGTATA GCTTTATCAC AAACTGTATA TTTTAAACTG TTAGCGACGT 1500
CCTTGGCCAC GAACCGGACC TGTTGGTCGC GCTCTAGCAC GTACCGCAGG TTGAACGTAT 1560
CTTCTCCAAA TTTAAATTCT CCAATTTTAA CGCGAGCCAT TTTGATACAC GTGTGTCGAT 1620
TTTGCAACAA CTATTGTTTT TTAACGCAAA CTAAACTTAT TGTGGTAAGC AATAATTAAA 1680
TATGGGGGAA CATGCGCCGC TACAACACTC GTCGTTATGA ACGCAGACGG CGCCGGTCTC 1740
GGCGCAAGCG GCTAAAACGT GTTGCGCGTT CAACGCGGCA AACATCGCAA AAGCCAATAG 1800
TACAGTTTTG ATTTGCATAT TAACGGCGAT TTTTTAAATT ATCTTATTTA ATAAATAGTT 1860
ATGACGCCTA CAACTCCCCG CCCGCGTTGA CTCGCTGCAC CTCGAGCAGT TCGTTGACGC 1920
CTTCCTCCGT GTGGCCGAAC ACGTCGAGCG GGTGGTCGAT GACCAGCGGC GTGCCGCACG 1980
CGACGCACAA GTATCTGTAC ACCGAATGAT CGTCGGGCGA AGGCACGTCG GCCTCCAAGT 2040
GGCAATATTG GCAAATTCGA AAATATATAC AGTTGGGTTG TTTGCGCATA TCTATCGTGG 2100
CGTTGGGCAT GTACGTCCGA ACGTTGATTT GCATGCAAGC CGAAATTAAA TCATTGCGAT 2160
TAGTGCGATT AAAACGTTGT ACATCCTCGC TTTTAATCAT GCCGTCGATT AAATCGCGCA 2220
ATCGAGTCAA GTGATCAAAG TGTGGAATAA TGTTTTCTTT GTATTCCCGA GTCAAGCGCA 2280
GCGCGTATTT TAACAAACTA GCCATCTTGT AAGTTAGTTT CATTTAATGC AACTTTATCC 2340
AATAATATAT TATGTATCGC ACGTCAAGAA TTAACAATGC GCCCGTTGTC GCATCTCAAC 2400
ACGACTATGA TAGAGATCAA ATAAAGCGCG AATTAAATAG CTTGCGACGC AACGTGCACG 2460
ATCTGTGCAC GCGTTCCGGC ACGAGCTTTG ATTGTAATAA GTTTTTACGA AGCGATGACA 2520
TGACCCCCGT AGTGACAACG ATCACGCCCA AAAGAACTGC CGACTACAAA ATTACCGAGT 2580
ATGTCGGTGA CGTTAAAACT ATTAAGCCAT CCAATCGACC GTTAGTCGAA TCAGGACCGC 2640
TGGTGCGAGA AGCCGCGAAG TATGGCGAAT GCATCGTATA ACGTGTGGAG TCCGCTCATT 2700
AGAGCGTCAT GTTTAGACAA GAAAGCTACA TATTTAATTG ATCCCGATGA TTTTATTGAT 2760

AAATTGACCC TAACTCCATA CACGGTATTC TACAATGGCG GGGTTTTGGT CAAAATTTCC 2820

GGACTGCGAT TGTACATGCT GTTAACGGCT CCGCCCACTA TTAATGAAAT TAAAAATTCC 2880

AATTTTAAAA AACGCAGCAA GAGAAACATT TGTATGAAAG AATGCGTAGA AGGAAAGAAA 2940

AATGTCGTCG ACATGCTGAA CAACAAGATT AATATGCCTC CGTGTATAAA AAAAATATTG 3000

AACGATTTGA AAGAAAACAA TGTACCGCGC GGCGGTATGT ACAGGAAGAG GTTTATACTA 3060

AACTGTTACA TTGCAAACGT GGTTTCGTGT GCCAAGTGTG AAAACCGATG TTTAATCAAG 3120

GCTCTGACGC ATTTCTACAA CCACGACTCC AAGTGTGTGG GTGAAGTCAT GCATCTTTTA 3180

ATCAAATCCC AAGATGTGTA TAAACCACCA AACTGCCAAA AAATGAAAAC TGTCGACAAG 3240

CTCTGTCCGT TTGCTGGCAA CTGCAAGGGT CTCAATCCTA TTTGTAATTA TTGAATAATA 3300

AAACAATTAT AAATGCTAAA TTTGTTTTTT ATTAACGATA CAAACCAAAC GCAACAAGAA 3360

CATTTGTAGT ATTATCTATA ATTGAAAACG CGTAGTTATA ATCGCTGAGG TAATATTTAA 3420

AATCATTTTC AAATGATTCA CAGTTAATTT GCGACAATAT AATTTTATTT TCACATAAAC 3480

TAGACGCCTT GTCGTCTTCT TCTTCGTATT CCTTCTCTTT TTCATTTTTC TCCTCATAAA 3540

AATTAACATA GTTATTATCG TATCCATATA TGTATCTATC GTATAGAGTA AATTTTTTGT 3600

TGTCATAAAT ATATATGTCT TTTTTAATGG GGTGTATAGT ACCGCTGCGC ATAGTTTTTC 3660

TGTAATTTAC AACAGTGCTA TTTTCTGGTA GTTCTTCGGA GTGTGTTGCT TTAATTATTA 3720

AATTTATATA ATCAATGAAT TTGGGATCGT CGGTTTTGTA CAATATGTTG CCGGCATAGT 3780

ACGCAGCTTC TTCTAGTTCA ATTACACCAT TTTTTAGCAG CACCGGATTA ACATAACTTT 3840

CCAAAATGTT GTACGAACCG TTAAACAAAA ACAGTTCACC TCCCTTTTCT ATACTATTGT 3900

CTGCGAGCAG TTGTTTGTTG TTAAAAATAA CAGCCATTGT AATGAGACGC ACAAACTAAT 3960

ATCACAAACT GGAAATGTCT ATCAATATAT AGTTGCTGAT ATCATGGAGA TAATTAAAAT 4020

GATAACCATC TCGCAAATAA ATAAGTATTT TACTGTTTTC GTAACAGTTT TGTAATAAAA 4080

AAACCTATAA ATATTCCGGA TTATTCATAC CGTCCCACCA TCGGGCGCGG ATCTATGCTA 4140

CTAGTAAATC AGTCACACCA AGGCTTCAAT AAGGAACACA CAAGCAAGAT GGTAAGCGCT 4200

ATTGTTTTAT ATGTGCTTTT GGCGGCGGCG GCGCATTCTG CCTTTGCGGA CTACAAGGAC 4260

GACGATGACA AGGGATCCCG GGCCATGGGA ATTCCGGAGC GGCCGCTGCA GATCTGATCC 4320

TTTCCTGGGA CCCGGCAAGA ACCAAAAACT CACTCTCTTC AAGGAAATCC GTAATGTTAA 4380

ACCCGACACG ATGAAGCTTG TCGTTGGATG GAAAGGAAAA GAGTTCTACA GGGAAACTTG 4440

GACCCGCTTC ATGGAAGACA GCTTCCCCAT TGTTAACGAC CAAGAAGTGA TGGATGTTTT 4500

CCTTGTTGTC AACATGCGTC CCACTAGACC CAACCGTTGT TACAAATTCC TGGCCCAACA 4560

CGCTCTGCGT TGCGACCCCG ACTATGTACC TCATGACGTG ATTAGGATCG TCGAGCCTTC 4620

ATGGGTGGGC AGCAACAACG AGTACCGCAT CAGCCTGGCT AAGAAGGGCG GCGGCTGCCC 4680

AATAATGAAC CTTCACTCTG AGTACACCAA CTCGTTCGAA CAGTTCATCG ATCGTGTCAT 4740
CTGGGAGAAC TTCTACAAGC CCATCGTTTA CATCGGTACC GACTCTGCTG AAGAGGAGGA 4800
AATTCTCCTT GAAGTTTCCC TGGTGTTCAA AGTAAAGGAG TTTGCACCAG ACGCACCTCT 4860
GTTCACTGGT CCGGCGTATT AAAACACGAT ACATTGTTAT TAGTACATTT ATTAAGCGCT 4920
AGATTCTGTG CGTTGTTGAT TTACAGACAA TTGTTGTACG TATTTTAATA ATTCATTAAA 4980
TTTATAATCT TTAGGGTGGT ATGTTAGAGC GAAAATCAAA TGATTTTCAG CGTCTTTATA 5040
TCTGAATTTA AATATTAAAT CCTCAATAGA TTTGTAAAAT AGGTTTCGAT TAGTTTCAAA 5100
CAAGGGTTGT TTTTCCGAAC CGATGGCTGG ACTATCTAAT GGATTTTCGC TCAACGCCAC 5160
AAAACTTGCC AAATCTTGTA GCAGCAATCT AGCTTTGTCG ATATTCGTTT GTGTTTTGTT 5220
TTGTAATAAA GGTTCGACGT CGTTCAAAAT ATTATGCGCT TTTGTATTTC TTTCATCACT 5280
GTCGTTAGTG TACAATTGAC TCGACGTAAA CACGTTAAAT AAAGCTTGGA CATATTTAAC 5340
ATCGGGCGTG TTAGCTTTAT TAGGCCGATT ATCGTCGTCG TCCCAACCCT CGTCGTTAGA 5400
AGTTGCTTCC GAAGACGATT TTGCCATAGC CACACGACGC CTATTAATTG TGTCGGCTAA 5460
CACGTCCGCG ATCAAATTTG TAGTTGAGCT TTTTGGAATT ATTTCTGATT GCGGGCGTTT 5520
TTGGGCGGGT TTCAATCTAA CTGTGCCCGA TTTTAATTCA GACAACACGT TAGAAAGCGA 5580
TGGTGCAGGC GGTGGTAACA TTTCAGACGG CAAATCTACT AATGGCGGCG GTGGTGGAGC 5640
TGATGATAAA TCTACCATCG GTGGAGGCGC AGGCGGGGCT GGCGGCGGAG GCGGAGGCGG 5700
AGGTGGTGGC GGTGATGCAG ACGGCGGTTT AGGCTCAAAT GTCTCTTTAG GCAACACAGT 5760
CGGCACCTCA ACTATTGTAC TGGTTTCGGG CGCCGTTTTT GGTTTGACCG GTCTGAGACG 5820
AGTGCGATTT TTTTCGTTTC TAATAGCTTC CAACAATTGT TGTCTGTCGT CTAAAGGTGC 5880
AGCGGGTTGA GGTTCCGTCG GCATTGGTGG AGCGGGCGGC AATTCAGACA TCGATGGTGG 5940
TGGTGGTGGT GGAGGCGCTG GAATGTTAGG CACGGGAGAA GGTGGTGGCG GCGGTGCCGC 6000
CGGTATAATT TGTTCTGGTT TAGTTTGTTC GCGCACGATT GTGGGCACCG GCGCAGGCGC 6060
CGCTGGCTGC ACAACGGAAG GTCGTCTGCT TCGAGGCAGC GCTTGGGGTG GTGGCAATTC 6120
AATATTATAA TTGGAATACA AATCGTAAAA ATCTGCTATA AGCATTGTAA TTTCGCTATC 6180
GTTTACCGTG CCGATATTTA ACAACCGCTC AATGTAAGCA ATTGTATTGT AAAGAGATTG 6240
TCTCAAGCTC CGCACGCCGA TAACAAGCCT TTTCATTTTT ACTACAGCAT TGTAGTGGCG 6300
AGACACTTCG CTGTCGTCGA CGTACATGTA TGCTTTGTTG TCAAAAACGT CGTTGGCAAG 6360
CTTTAAAATA TTTAAAAGAA CATCTCTGTT CAGCACCACT GTGTTGTCGT AAATGTTGTT 6420
TTTGATAATT TGCGCTTCCG CAGTATCGAC ACGTTCAAAA AATTGATGCG CATCAATTTT 6480
GTTGTTCCTA TTATTGAATA AATAAGATTG TACAGATTCA TATCTACGAT TCGTCATGGC 6540

CACCACAAAT GCTACGCTGC AAACGCTGGT ACAATTTTAC GAAAACTGCA AAAACGTCAA 6600
AACTCGGTAT AAAATAATCA ACGGGCGCTT TGGCAAAATA TCTATTTTAT CGCACAAGCC 6660
CACTAGCAAA TTGTATTTGC AGAAAACAAT TTCGGCGCAC AATTTTAACG CTGACGAAAT 6720
AAAAGTTCAC CAGTTAATGA GCGACCACCC AAATTTTATA AAAATCTATT TTAATCACGG 6780
TTCCATCAAC AACCAAGTGA TCGTGATGGA CTACATTGAC TGTCCCGATT TATTTGAAAC 6840
ACTACAAATT AAAGGCGAGC TTTCGTACCA ACTTGTTAGC AATATTATTA GACAGCTGTG 6900
TGAAGCGCTC AACGATTTGC ACAAGCACAA TTTCATACAC AACGACATAA AACTCGAAAA 6960
TGTCTTATAT TTCGAAGCAC TTGATCGCGT GTATGTTTGC GATTACGGAT TGTGCAAACA 7020
CGAAAACTCA CTTAGCGTGC ACGACGGCAC GTTGGAGTAT TTTAGTCCGG AAAAAATTCG 7080
ACACACAACT ATGCACGTTT CGTTTGACTG GTACGCGGCG TGTTAACATA CAAGTTGCTA 7140
ACCGGCGGTT CGTAATCATG GTCATAGCTG TTTCCTGTGT GAAATTGTTA TCCGCTCACA 7200
ATTCCACACA ACATACGAGC CGGAAGCATA AAGTGTAAAG CCTGGGGTGC CTAATGAGTG 7260
AGCTAACTCA CATTAATTGC GTTGCGCTCA CTGCCCGCTT TCCAGTCGGG AAACCTGTCG 7320
TGCCAGCTGC ATTAATGAAT CGGCCAACGC GCGGGGAGAG GCGGTTTGCG TATTGGGCGC 7380
TCTTCCGCTT CCTCGCTCAC TGACTCGCTG CGCTCGGTCG TTCGGCTGCG GCGAGCGGTA 7440
TCAGCTCACT CAAAGGCGGT AATACGGTTA TCCACAGAAT CAGGGGATAA CGCAGGAAAG 7500
AACATGTGAG CAAAAGGCCA GCAAAAGGCC AGGAACCGTA AAAAGGCCGC GTTGCTGGCG 7560
TTTTTCCATA GGCTCCGCCC CCCTGACGAG CATCACAAAA ATCGACGCTC AAGTCAGAGG 7620
TGGCGAAACC CGACAGGACT ATAAAGATAC CAGGCGTTTC CCCCTGGAAG CTCCCTCGTG 7680
CGCTCTCCTG TTCCGACCCT GCCGCTTACC GGATACCTGT CCGCCTTTCT CCCTTCGGGA 7740
AGCGTGGCGC TTTCTCATAG CTCACGCTGT AGGTATCTCA GTTCGGTGTA GGTCGTTCGC 7800
TCCAAGCTGG GCTGTGTGCA CGAACCCCCC GTTCAGCCCG ACCGCTGCGC CTTATCCGGT 7860
AACTATCGTC TTGAGTCCAA CCCGGTAAGA CACGACTTAT CGCCACTGGC AGCAGCCACT 7920
GGTAACAGGA TTAGCAGAGC GAGGTATGTA GGCGGTGCTA CAGAGTTCTT GAAGTGGTGG 7980
CCTAACTACG GCTACACTAG AAGGACAGTA TTTGGTATCT GCGCTCTGCT GAAGCCAGTT 8040
ACCTTCGGAA AAAGAGTTGG TAGCTCTTGA TCCGGCAAAC AAACCACCGC TGGTAGCGGT 8100
GGTTTTTTTG TTTGCAAGCA GCAGATTACG CGCAGAAAAA AAGGATCTCA AGAAGATCCT 8160
TTGATCTTTT CTACGGGGTC TGACGCTCAG TGGAACGAAA ACTCACGTTA AGGGATTTTG 8220
GTCATGAGAT TATCAAAAAG GATCTTCACC TAGATCCTTT TAAATTAAAA ATGAAGTTTT 8280
AAATCAATCT AAAGTATATA TGAGTAAACT TGGTCTGACA GTTACCAATG CTTAATCAGT 8340
GAGGCACCTA TCTCAGCGAT CTGTCTATTT CGTTCATCCA TAGTTGCCTG ACTCCCCGTC 8400
GTGTAGATAA CTACGATACG GGAGGGCTTA CCATCTGGCC CCAGTGCTGC AATGATACCG 8460

CGAGACCCAC GCTCACCGGC TCCAGATTTA TCAGCAATAA ACCAGCCAGC CGGAAGGGCC 8520

GAGCGCAGAA GTGGTCCTGC AACTTTATCC GCCTCCATCC AGTCTATTAA TTGTTGCCGG 8580

GAAGCTAGAG TAAGTAGTTC GCCAGTTAAT AGTTTGCGCA ACGTTGTTGC CATTGCTACA 8640

GGCATCGTGG TGTCACGCTC GTCGTTTGGT ATGGCTTCAT TCAGCTCCGG TTCCCAACGA 8700

TCAAGGCGAG TTACATGATC CCCCATGTTG TGCAAAAAAG CGGTTAGCTC CTTCGGTCCT 8760

CCGATCGTTG TCAGAAGTAA GTTGGCCGCA GTGTTATCAC TCATGGTTAT GGCAGCACTG 8820

CATAATTCTC TTACTGTCAT GCCATCCGTA AGATGCTTTT CTGTGACTGG TGAGTACTCA 8880

ACCAAGTCAT TCTGAGAATA GTGTATGCGG CGACCGAGTT GCTCTTGCCC GGCGTCAATA 8940

CGGGATAATA CCGCGCCACA TAGCAGAACT TTAAAAGTGC TCATCATTGG AAAACGTTCT 9000

TCGGGGCGAA AACTCTCAAG GATCTTACCG CTGTTGAGAT CCAGTTCGAT GTAACCCACT 9060

CGTGCACCCA ACTGATCTTC AGCATCTTTT ACTTTCACCA GCGTTTCTGG GTGAGCAAAA 9120

ACAGGAAGGC AAAATGCCGC AAAAAAGGGA ATAAGGGCGA CACGGAAATG TTGAATACTC 9180

ATACTCTTCC TTTTTCAATA TTATTGAAGC ATTTATCAGG GTTATTGTCT CATGAGCGGA 9240

TACATATTTG AATGTATTTA GAAAAATAAA CAAATAGGGG TTCCGCGCAC ATTTCCCCGA 9300

AAAGTGCCAC CTGACGTCTA AGAAACCATT ATTATCATGA CATTAACCTA TAAAAATAGG 9360

CGTATCACGA GGCCCTTTCG TCTCGCGCGT TTCGGTGATG ACGGTGAAAA CCTCTGACAC 9420

ATGCAGCTCC CGGAGACGGT CACAGCTTGT CTGTAAGCGG ATGCCGGGAG CAGACAAGCC 9480

CGTCAGGGCG CGTCAGCGGG TGTTGGCGGG TGTCGGGGCT GGCTTAACTA TGCGGCATCA 9540

GAGCAGATTG TACTGAGAGT GCACCATATG CGGTGTGAAA TACCGCACAG ATGCGTAAGG 9600

AGAAAATACC GCATCAGGCG CCATTCGCCA TTCAGGCTGC GCAACTGTTG GGAAGGGCGA 9660

TCGGTGCGGG CCTCTTCGCT ATTACGCCAG CTGGCGAAAG GGGGATGTGC TGCAAGGCGA 9720

TTAAGTTGGG TAACGCCAGG GTTTTCCCAG TCACGACGTT GTAAAACGAC GGCCAGTGCC 9780

### Annex to the application documents - subsequently filed sequences listing

SEQUENCE LISTING

<110> Weiss, Stefan

<120> Method of diagnosis of transmissible spongiform
      encephalopathy (TSE)

<130> 11503/CH

<140> EP 01108876.2
<141> 2001-04-09

<150> EP 97108712.7
<151> 1997-05-30

<160> 3

<170> PatentIn Ver. 2.1

<210> 1
<211> 24
<212> DNA
<213> Artificial Sequence

<220>
<223> Description of Artificial Sequence:
      oligodeoxyribonucleotide

<400> 1
ctatgctact agtaaatcag tcac                                        24

<210> 2
<211> 59
<212> DNA
<213> Artificial Sequence

<220>
<223> Description of Artificial Sequence:
      oligodeoxyribonucleotide

<400> 2
cccatggccc gggatccctt gtcatcgtcg tccttgtagt ccgcaaaggc agaatgcgc  59

<210> 3
<211> 9780
<212> DNA

<213> Artificial Sequence

<220>
<223> Description of Artificial Sequence:baculovirus
      transfer vector pFLAG-BAC

<400> 3
aagctttact cgtaaagcga gttgaaggat catatttagt tgcgtttatg agataagatt 60
gaaagcacgt gtaaaatgtt tcccgcgcgt tggcacaact atttacaatg cggccaagtt 120
ataaaagatt ctaatctgat atgttttaaa acacctttgc ggcccgagtt gtttgcgtac 180
gtgactagcg aagaagatgt gtggaccgca gaacagatag taaaacaaaa ccctagtatt 240
ggagcaataa tcgatttaac caacacgtct aaatattatg atggtgtgca ttttttgcgg 300
gcgggcctgt tatacaaaaa aattcaagta cctggccaga ctttgccgcc tgaaagcata 360
gttcaagaat ttattgacac ggtaaaagaa tttacagaaa agtgtcccgg catgttggtg 420
ggcgtgcact gcacacacgg tattaatcgc accggttaca tggtgtgcag atatttaatg 480
cacaccctgg gtattgcgcc gcaggaagcc atagatagat tcgaaaaagc cagaggtcac 540
aaaattgaaa gacaaaatta cgttcaagat ttattaattt aattaatatt atttgcattc 600
tttaacaaat actttatcct attttcaaat tgttgcgctt cttccagcga accaaaacta 660
tgcttcgctt gctccgttta gcttgtagcc gatcagtggc gttgttccaa tcgacggtag 720
gattaggccg gatattctcc accacaatgt tggcaacgtt gatgttacgt ttatgctttt 780
ggttttccac gtacgtcttt tggccggtaa tagccgtaaa cgtagtgccg tcgcgcgtca 840
cgcacaacac cggatgtttg cgcttgtccg cggggtattg aaccgcgcga tccgacaaat 900
ccaccacttt ggcaactaaa tcggtgacct gcgcgtcttt tttctgcatt atttcgtctt 960
tcttttgcat ggtttcctgg aagccggtgt acatgcggtt tagatcagtc atgacgcgcg 1020
tgacctgcaa atctttggcc tcgatctgct tgtccttgat ggcaacgatg cgttcaataa 1080
actcttgttt tttaacaagt tcctcggttt tttgcgccac caccgcttgc agcgcgtttg 1140
tgtgctcggt gaatgtcgca atcagcttag tcaccaactg tttgctctcc tcctcccgtt 1200
gtttgatcgc gggatcgtac ttgccggtgc agagcacttg aggaattact tcttctaaaa 1260
gccattcttg taattctatg gcgtaaggca atttggactt cataatcagc tgaatcacgc 1320
cggatttagt aatgagcact gtatgcggct gcaaatacag cgggtcgccc cttttcacga 1380
cgctgttaga ggtagggccc ccattttgga tggtctgctc aaataacgat ttgtatttat 1440
tgtctacatg aacacgtata gctttatcac aaactgtata ttttaaactg ttagcgacgt 1500
ccttggccac gaaccggacc tgttggtcgc gctctagcac gtaccgcagg ttgaacgtat 1560
cttctccaaa tttaaattct ccaattttaa cgcgagccat tttgatacac gtgtgtcgat 1620
tttgcaacaa ctattgtttt ttaacgcaaa ctaaacttat tgtggtaagc aataattaaa 1680
tatgggggaa catgcgccgc tacaacactc gtcgttatga acgcagacgg cgccggtctc 1740
ggcgcaagcg gctaaaacgt gttgcgcgtt caacgcggca aacatcgcaa aagccaatag 1800
tacagttttg atttgcatat taacggcgat tttttaaatt atcttattta ataaatagtt 1860
atgacgccta caactccccg cccgcgttga ctcgctgcac ctcgagcagt tcgttgacgc 1920
cttcctccgt gtggccgaac acgtcgagcg ggtggtcgat gaccagcggc gtgccgcacg 1980
cgacgcacaa gtatctgtac accgaatgat cgtcgggcga aggcacgtcg gcctccaagt 2040
ggcaatattg gcaaattcga aaatatatac agttgggttg tttgcgcata tctatcgtgg 2100
cgttgggcat gtacgtccga acgttgattt gcatgcaagc cgaaattaaa tcattgcgat 2160
tagtgcgatt aaaacgttgt acatcctcgc ttttaatcat gccgtcgatt aaatcgcgca 2220
atcgagtcaa gtgatcaaag tgtggaataa tgttttcttt gtattcccga gtcaagcgca 2280
gcgcgtattt taacaaacta gccatcttgt aagttagttt catttaatgc aactttatcc 2340
aataatatat tatgtatcgc acgtcaagaa ttaacaatgc gcccgttgtc gcatctcaac 2400
acgactatga tagagatcaa ataaagcgcg aattaaatag cttgcgacgc aacgtgcacg 2460

atctgtgcac gcgttccggc acgagctttg attgtaataa gtttttacga agcgatgaca 2520
tgacccccgt agtgacaacg atcacgccca aaagaactgc cgactacaaa attaccgagt 2580
atgtcggtga cgttaaaact attaagccat ccaatcgacc gttagtcgaa tcaggaccgc 2640
tggtgcgaga agccgcgaag tatggcgaat gcatcgtata acgtgtggag tccgctcatt 2700
agagcgtcat gtttagacaa gaaagctaca tatttaattg atcccgatga ttttattgat 2760
aaattgaccc taactccata cacggtattc tacaatggcg gggttttggt caaaatttcc 2820
ggactgcgat tgtacatgct gttaacggct ccgcccacta ttaatgaaat taaaaattcc 2880
aattttaaaa aacgcagcaa gagaaacatt tgtatgaaag aatgcgtaga aggaaagaaa 2940
aatgtcgtcg acatgctgaa caacaagatt aatatgcctc cgtgtataaa aaaaatattg 3000
aacgatttga aagaaaacaa tgtaccgcgc ggcggtatgt acaggaagag gtttatacta 3060
aactgttaca ttgcaaacgt ggtttcgtgt gccaagtgtg aaaaccgatg tttaatcaag 3120
gctctgacgc atttctacaa ccacgactcc aagtgtgtgg gtgaagtcat gcatctttta 3180
atcaaatccc aagatgtgta taaaccacca aactgccaaa aaatgaaaac tgtcgacaag 3240
ctctgtccgt ttgctggcaa ctgcaagggt ctcaatccta tttgtaatta ttgaataata 3300
aaacaattat aaatgctaaa tttgtttttt attaacgata caaaccaaac gcaacaagaa 3360
catttgtagt attatctata attgaaaacg cgtagttata atcgctgagg taatatttaa 3420
aatcattttc aaatgattca cagttaattt gcgacaatat aattttattt tcacataaac 3480
tagacgcctt gtcgtcttct tcttcgtatt ccttctcttt ttcatttttc tcctcataaa 3540
aattaacata gttattatcg tatccatata tgtatctatc gtatagagta aattttttgt 3600
tgtcataaat atatatgtct tttttaatgg ggtgtatagt accgctgcgc atagtttttc 3660
tgtaatttac aacagtgcta ttttctggta gttcttcgga gtgtgttgct ttaattatta 3720
aatttatata atcaatgaat ttgggatcgt cggttttgta caatatgttg ccggcatagt 3780
acgcagcttc ttctagttca attacaccat tttttagcag caccggatta acataacttt 3840
ccaaaatgtt gtacgaaccg ttaaacaaaa acagttcacc tcccttttct atactattgt 3900
ctgcgagcag ttgtttgttg ttaaaaataa cagccattgt aatgagacgc acaaactaat 3960
atcacaaact ggaaatgtct atcaatatat agttgctgat atcatggaga taattaaaat 4020
gataaccatc tcgcaaataa ataagtattt tactgttttc gtaacagttt tgtaataaaa 4080
aaacctataa atattccgga ttattcatac cgtcccacca tcgggcgcgg atctatgcta 4140
ctagtaaatc agtcacacca aggcttcaat aaggaacaca caagcaagat ggtaagcgct 4200
attgttttat atgtgctttt ggcggcggcg gcgcattctg cctttgcgga ctacaaggac 4260
gacgatgaca agggatcccg ggccatggga attccggagc ggccgctgca gatctgatcc 4320
tttcctggga cccggcaaga accaaaaact cactctcttc aaggaaatcc gtaatgttaa 4380
acccgacacg atgaagcttg tcgttggatg gaaaggaaaa gagttctaca gggaaacttg 4440
gacccgcttc atggaagaca gcttccccat tgttaacgac caagaagtga tggatgtttt 4500
ccttgttgtc aacatgcgtc ccactagacc caaccgttgt tacaaattcc tggcccaaca 4560
cgctctgcgt tgcgaccccg actatgtacc tcatgacgtg attaggatcg tcgagccttc 4620
atgggtgggc agcaacaacg agtaccgcat cagcctggct aagaagggcg gcggctgccc 4680
aataatgaac cttcactctg agtacaccaa ctcgttcgaa cagttcatcg atcgtgtcat 4740
ctgggagaac ttctacaagc ccatcgttta catcggtacc gactctgctg aagaggagga 4800
aattctcctt gaagtttccc tggtgttcaa agtaaaggag tttgcaccag acgcacctct 4860
gttcactggt ccggcgtatt aaaacacgat acattgttat tagtacattt attaagcgct 4920
agattctgtg cgttgttgat ttacagacaa ttgttgtacg tattttaata attcattaaa 4980
tttataatct ttagggtggt atgttagagc gaaaatcaaa tgattttcag cgtctttata 5040
tctgaattta aatattaaat cctcaataga tttgtaaaat aggtttcgat tagtttcaaa 5100
caagggttgt ttttccgaac cgatggctgg actatctaat ggattttcgc tcaacgccac 5160
aaaacttgcc aaatcttgta gcagcaatct agctttgtcg atattcgttt gtgttttgtt 5220
ttgtaataaa ggttcgacgt cgttcaaaat attatgcgct tttgtatttc tttcatcact 5280
gtcgttagtg tacaattgac tcgacgtaaa cacgttaaat aaagcttgga catatttaac 5340

atcgggcgtg ttagctttat taggccgatt atcgtcgtcg tcccaaccct cgtcgttaga 5400
agttgcttcc gaagacgatt ttgccatagc cacacgacgc ctattaattg tgtcggctaa 5460
cacgtccgcg atcaaatttg tagttgagct ttttggaatt atttctgatt gcgggcgttt 5520
ttgggcgggt ttcaatctaa ctgtgcccga ttttaattca gacaacacgt tagaaagcga 5580
tggtgcaggc ggtggtaaca tttcagacgg caaatctact aatggcggcg gtggtggagc 5640
tgatgataaa tctaccatcg gtggaggcgc aggcggggct ggcggcggag gcggaggcgg 5700
aggtggtggc ggtgatgcag acggcggttt aggctcaaat gtctctttag gcaacacagt 5760
cggcacctca actattgtac tggtttcggg cgccgttttt ggtttgaccg gtctgagacg 5820
agtgcgattt ttttcgtttc taatagcttc caacaattgt tgtctgtcgt ctaaaggtgc 5880
agcgggttga ggttccgtcg gcattggtgg agcgggcggc aattcagaca tcgatggtgg 5940
tggtggtggt ggaggcgctg gaatgttagg cacgggagaa ggtggtggcg gcggtgccgc 6000
cggtataatt tgttctggtt tagtttgttc gcgcacgatt gtgggcaccg gcgcaggcgc 6060
cgctggctgc acaacggaag gtcgtctgct tcgaggcagc gcttggggtg gtggcaattc 6120
aatattataa ttggaataca aatcgtaaaa atctgctata agcattgtaa tttcgctatc 6180
gtttaccgtg ccgatattta acaaccgctc aatgtaagca attgtattgt aaagagattg 6240
tctcaagctc cgcacgccga taacaagcct tttcattttt actacagcat tgtagtggcg 6300
agacacttcg ctgtcgtcga cgtacatgta tgctttgttg tcaaaaacgt cgttggcaag 6360
ctttaaaata tttaaaagaa catctctgtt cagcaccact gtgttgtcgt aaatgttgtt 6420
tttgataatt tgcgcttccg cagtatcgac acgttcaaaa aattgatgcg catcaatttt 6480
gttgttccta ttattgaata aataagattg tacagattca tatctacgat tcgtcatggc 6540
caccacaaat gctacgctgc aaacgctggt acaattttac gaaaactgca aaaacgtcaa 6600
aactcggtat aaaataatca acgggcgctt tggcaaaata tctattttat cgcacaagcc 6660
cactagcaaa ttgtatttgc agaaaacaat ttcggcgcac aattttaacg ctgacgaaat 6720
aaaagttcac cagttaatga gcgaccaccc aaattttata aaaatctatt ttaatcacgg 6780
ttccatcaac aaccaagtga tcgtgatgga ctacattgac tgtcccgatt tatttgaaac 6840
actacaaatt aaaggcgagc tttcgtacca acttgttagc aatattatta gacagctgtg 6900
tgaagcgctc aacgatttgc acaagcacaa tttcatacac aacgacataa aactcgaaaa 6960
tgtcttatat ttcgaagcac ttgatcgcgt gtatgtttgc gattacggat tgtgcaaaca 7020
cgaaaactca cttagcgtgc acgacggcac gttggagtat tttagtccgg aaaaaattcg 7080
acacacaact atgcacgttt cgtttgactg gtacgcggcg tgttaacata caagttgcta 7140
accggcggtt cgtaatcatg gtcatagctg tttcctgtgt gaaattgtta tccgctcaca 7200
attccacaca acatacgagc cggaagcata aagtgtaaag cctggggtgc ctaatgagtg 7260
agctaactca cattaattgc gttgcgctca ctgcccgctt tccagtcggg aaacctgtcg 7320
tgccagctgc attaatgaat cggccaacgc gcggggagag gcggtttgcg tattgggcgc 7380
tcttccgctt cctcgctcac tgactcgctg cgctcggtcg ttcggctgcg gcgagcggta 7440
tcagctcact caaaggcggt aatacggtta tccacagaat caggggataa cgcaggaaag 7500
aacatgtgag caaaaggcca gcaaaaggcc aggaaccgta aaaaggccgc gttgctggcg 7560
tttttccata ggctccgccc ccctgacgag catcacaaaa atcgacgctc aagtcagagg 7620
tggcgaaacc cgacaggact ataaagatac caggcgtttc cccctggaag ctccctcgtg 7680
cgctctcctg ttccgaccct gccgcttacc ggatacctgt ccgcctttct cccttcggga 7740
agcgtggcgc tttctcatag ctcacgctgt aggtatctca gttcggtgta ggtcgttcgc 7800
tccaagctgg gctgtgtgca cgaacccccc gttcagcccg accgctgcgc cttatccggt 7860
aactatcgtc ttgagtccaa cccggtaaga cacgacttat cgccactggc agcagccact 7920
ggtaacagga ttagcagagc gaggtatgta ggcggtgcta cagagttctt gaagtggtgg 7980
cctaactacg gctacactag aaggacagta tttggtatct gcgctctgct gaagccagtt 8040
accttcggaa aaagagttgg tagctcttga tccggcaaac aaaccaccgc tggtagcggt 8100
ggtttttttg tttgcaagca gcagattacg cgcagaaaaa aaggatctca agaagatcct 8160
ttgatctttt ctacggggtc tgacgctcag tggaacgaaa actcacgtta agggattttg 8220

gtcatgagat tatcaaaaag gatcttcacc tagatccttt taaattaaaa atgaagtttt 8280
aaatcaatct aaagtatata tgagtaaact tggtctgaca gttaccaatg cttaatcagt 8340
gaggcaccta tctcagcgat ctgtctattt cgttcatcca tagttgcctg actccccgtc 8400
gtgtagataa ctacgatacg ggagggctta ccatctggcc ccagtgctgc aatgataccg 8460
cgagacccac gctcaccggc tccagattta tcagcaataa accagccagc cggaagggcc 8520
gagcgcagaa gtggtcctgc aactttatcc gcctccatcc agtctattaa ttgttgccgg 8580
gaagctagag taagtagttc gccagttaat agtttgcgca acgttgttgc cattgctaca 8640
ggcatcgtgg tgtcacgctc gtcgtttggt atggcttcat tcagctccgg ttcccaacga 8700
tcaaggcgag ttacatgatc ccccatgttg tgcaaaaaag cggttagctc cttcggtcct 8760
ccgatcgttg tcagaagtaa gttggccgca gtgttatcac tcatggttat ggcagcactg 8820
cataattctc ttactgtcat gccatccgta agatgctttt ctgtgactgg tgagtactca 8880
accaagtcat tctgagaata gtgtatgcgg cgaccgagtt gctcttgccc ggcgtcaata 8940
cgggataata ccgcgccaca tagcagaact ttaaaagtgc tcatcattgg aaaacgttct 9000
tcggggcgaa aactctcaag gatcttaccg ctgttgagat ccagttcgat gtaacccact 9060
cgtgcaccca actgatcttc agcatctttt actttcacca gcgtttctgg gtgagcaaaa 9120
acaggaaggc aaaatgccgc aaaaaaggga ataagggcga cacggaaatg ttgaatactc 9180
atactcttcc tttttcaata ttattgaagc atttatcagg gttattgtct catgagcgga 9240
tacatatttg aatgtattta gaaaaataaa caaatagggg ttccgcgcac atttccccga 9300
aaagtgccac ctgacgtcta agaaaccatt attatcatga cattaaccta taaaaatagg 9360
cgtatcacga ggccctttcg tctcgcgcgt ttcggtgatg acggtgaaaa cctctgacac 9420
atgcagctcc cggagacggt cacagcttgt ctgtaagcgg atgccgggag cagacaagcc 9480
cgtcagggcg cgtcagcggg tgttggcggg tgtcggggct ggcttaacta tgcggcatca 9540
gagcagattg tactgagagt gcaccatatg cggtgtgaaa taccgcacag atgcgtaagg 9600
agaaaatacc gcatcaggcg ccattcgcca ttcaggctgc gcaactgttg ggaagggcga 9660
tcggtgcggg cctcttcgct attacgccag ctggcgaaag ggggatgtgc tgcaaggcga 9720
ttaagttggg taacgccagg gttttcccag tcacgacgtt gtaaaacgac ggccagtgcc 9780

## Description

The present invention relates to pharmaceutical compositions comprising a soluble laminin receptor precursor (LRP) or a modification thereof. The present invention also relates to pharmaceutical compositions comprising a compound which blocks the interaction of LRP and PrP^{Sc} or PrP^{c}. Said compositions allow the treatment of diseases associated with the presence of PrP^{Sc}. In addition, the present invention provides an expression vector for the recombinant production of LRP or any kind of protein in the baculovirus system.

Prion proteins are thought to play a central role in the pathogenesis of transmissible spongiform encephalopathies, such as bovine spongiform encephalopathy (BSE), Creutzfeldt-Jakob disease (CJD) and Scrapie (Prusiner, 1991). This unique group of neurodegenerative disorders can be inherited due to known mutations in the chromosomal prion protein gene, they can originate sporadically and can be caused by transmission of infectious particles prepared from organs of affected organisms (for review see Weissmann et al., 1994). The transmissible agent seems to be an infectious protein (PrP^{Sc}) capable of replicating in the host in the absence of any nucleic acid (Prusiner et al., 1982; Alper et al., 1967; Griffith 1967), although recent transmission studies of the BSE agent to mice suggest that PrP^{Sc} might be involved in species adaptation, but that an unidentified agent may actually transmit BSE (Lasmezas et al., 1997). PrP^{Sc}, a pathogenic isoform of the normal host-encoded PrP^{c}, is thought to convert host-PrP^{c} into PrP^{Sc} resulting in pathological modifications in the brain such as spongiform vacuolation, astrocytic proliferation and neuronal loss.
PrP^{c} and PrP^{Sc} are identical regarding primary structure (Stahl et al., 1993), but CD-and FTIR-spectroscopy reveals that they have different secondary structures (Meyer et al., 1986; Pan et al., 1993). PrP^{c} has a very low content and PrP^{Sc} a high content of β-sheet structure. Moreover, PrP^{c} can be hydrolyzed completely by proteinase K, whereas PrP^{Sc} is degraded to the proteinase K resistant infectious core PrP27-30 (Caughey *et al.,* 1991; Prusiner et al., 1981; 1983; 1984). A proteinase K sensitive, soluble and presumably non-infectious version of PrP27-30, designated as sPrP27-30 was identified in human platelets (Perini et al., 1996) and might also act as a precursor for proteinase K resistant PrP27-30.

The conversion of PrP^{c} into PrP^{Sc} is thought to involve structural rearrangements of the polypeptide from α-helix to β-sheet. Two models are currently used to describe this conversion mechanism. According to the heterodimer model (Prusiner, 1982) one exogenous PrP^{Sc} molecule contacts host PrP^{c} resulting in a PrP^{c}-PrP^{Sc} heterodimer which refolds into a PrP^{Sc}-homodimer. In contrast, the nucleation dependent polymerization model (Lansbury and Caughey, 1995) proposes that infectivity is a property of a PrP^{Sc} oligomer, which can act as a seed for polymerization.
The physiological role of the cellular prion protein PrP^{c} was investigated with a series of knock-out experiments leading to controversial results. Most of the researchers did not recognize any changes in the behaviour or reproduction of PrP-knock-out mice (Büehler et al., 1992). More recent investigations with PrP^{o/o} mice suggested an important role of PrP^{c} in synaptic function including an efficient GABA_{A} receptor-mediated fast inhibition (Collinge et al., 1996) whereas another study did not find any dysfunctions of synapses in the hippocampus (Lledo et al., 1996). These discrepancies in the observation of phenotypes in PrP^{o/o} mouse lines may be due to the genetically heterogeneous background (Borchelt and Sisodia, 1996) and the different age of the animals. In summary, the physiological role of PrP^{c} remains unclear. However, PrP^{o/o} mice lacking the *prn-p* gene are, in contrast to wild-type mice, resistant to infections with the mouse PrP^{Sc} (Büehler et al., 1993) demonstrating that PrP^{Sc} is essential for the development of Scrapie and Scrapie-like disease. More recent experiments demonstrated that PrP^{c} is necessary for scrapie induced neurotoxicity (Brandner et al., 1996) and required for scrapie spread within the central nervous system (Brandner et al., 1996b).

So far, a receptor or coreceptors for prion-protein, which might be involved in the proliferation of PrP^{Sc} could not be identified. In addition, no method exists for the treatment of the above cited diseases which are related to the presence of PrP^{Sc}.

Thus, the technical problem underlying the present invention is to provide compounds which can be used for the treatment of diseases which are associated with the presence of PrP^{Sc}.

The solution to said technical problem is achieved by providing the embodiments characterized in the claims.

It has now been surprisingly and unexpectedly found that the laminin receptor precursor (LRP) specifically interacts with PrP^{c} and PrP^{Sc}.

In order to enlighten the normal biological function of PrP^{c} and to search for additional cellular factors involved in prion replication, a yeast two hybrid screen was initiated and molecular chaperones of the Hsp60 family as specific interactors for PrP^{c} could be identified (Edenhofer et al., 1996). In a more detailed analysis of the interactors of this screen, the 67 kDa laminin receptor precursor (LRP) was identified as an interactor for PrP^{c} (PrP23-231) or PrP^{Sc} fused to GST. The LRP/PrP interaction site was mapped. The specificity of this interaction was confirmed in insect and mammalian cells which have been co-infected with recombinant baculoviruses or co-transfected with plasmids, respectively, leading to a simultaneous expression of one of the prion proteins (PrP) and the laminin receptor precursor (LRP). The LRP/PrP interaction might be involved in proliferation of PrP^{Sc} and, thus, by blocking this interaction, diseases associated with the presence of PrP^{Sc} can be treated.

In the present invention, a specific interaction between the prion protein PrP^{c} and the 67 kDa laminin receptor precursor (LRP) is reported for the first time. The receptor was identified in the yeast two-hybrid system employing a LexA-GST::PrP^{c} fusion as the bait protein. Six different cDNAs have been isolated which encode the 67 kDa Laminin receptor precursor (LRP, Acc. No.10-2029) lacking 43, 45, 46, 89, 130 and 156 amino acids (the latter containing 23 amino acids not homologous to the laminin receptor precursor), respectively, at the amino terminus. The specificity of this interaction was confirmed by retransformation experiments employing bait plasmids encoding LexA-GST::PrP^{c}, LexA-GST, LexA and the false baits LexA-CTF2 (Wendler et al., 1994) and LexA-bicoid (Gyuris et al., 1993). Only LexA-GST::PrP^{c} bound to LRP, whereas none of the false baits interacted with the receptor. Expression of the LexA-GST::PrP^{c} bait was confirmed by using a PrP-specific antibody. Repressor assays with lexA-GST::PrP^{c} demonstrated that PrP^{c} did not change the binding characteristics of the lexA binding domain.
To map the LRP binding site for PrP, prey plasmids lacking 43 amino acids up to 179 amino acids at the amino terminal end of the laminin receptor precursor were retransformed with lexA-GST::PrP^{c}. Prey LRPΔ157 did interact with PrP whereas LRPΔ180 failed to bind to the prion protein demonstrating that the laminin binding domain (amino acid 161 to amino acid 180; Castronovo et al., 1991) and the PrP binding domain are identical.
In order to confirm the PrP/LRP interaction in another eukaryotic cell system, LRP (amino acid 44-amino acid 295) was expressed in baculovirus infected insect cells as a fusion with GST. Although the signal sequence of gp67 was present at the N-terminus, GST::LRP was not secreted into the culture medium but synthesized cell-associated most likely due to the presence of a short transmembrane region which was calculated between amino acid 86 and amino acid 101 of LRP (Castronovo et al., 1991a). GST::LRP which was specifically recognized by a LRP specific antibody revealed a molecular weight of 67 kDa. A polyclonal antibody directed against GST::LRP recognized recombinant LRP but revealed some background reactivity to GST. After thrombin cleavage of the fusion protein SDS gel analysis confirmed molecular weights of 37 kDa for the receptor and 27 kDa for GST. The cDNA encoding the laminin receptor precursor was initially isolated in attempts to identify the gene for the 67 kDa laminin receptor precursor (Yow et al., 1988). The cDNA, however, encoded for a 37 kDa protein (Yow et al., 1988), whereas the receptor was isolated as a 67 kDa protein from solid tumors (Malinoff and Wicha, 1983; Lesot et al., 1983; Rao et al., 1983). Pulse-Chase experiments suggest that the 37 kDa receptor could be the precursor of the 67 kDa receptor (Castronovo et al., 1991b). Homodimerization (Landowski et al., 1995) or the existence of an unknown factor (Castronovo et al., 1991b) could account for the 37/67 kDa polymorphism of the laminin receptor precursor.

Several classes of laminin binding proteins have been described including integrins (Albelda and Buck, 1990) and galactoside binding lectins. The non-integrin binding 67 kD high affinity laminin receptor precursor (LRP) binds beside laminin (Mecham et al., 1989) also elastin (Hinek et al., 1988). In order to prove the laminin binding ability of recombinant LRP fused to GST, binding assays in the presence of laminin were performed. Laminin represents a glycoprotein of the extracellular matrix (ECM) mediating cell attachment, differentiation, movement and growth (Hunter et *al.,* 1989). Laminin was isolated from murine Engelbreth-Holm-Swarm (EHS) tumors and parietal yolk sac (PYS) carcinoma cells (for review see Mecham, 1991). For the binding assays EHS laminin was used which consists of three polypeptide chains - A or α (440 kDa), B1 or β, and B2 or γ (each 220 kDa) - linked via disulfide bridges resulting in the typical cross-structure (Beck et al., 1990; Burgeson et al., 1994). Specific EHS-laminin binding to the receptor was demonstrated in the presence of GST::LRP whereas no binding occurred with GST. The Laminin/LRP interaction can be competed with FLAG::rPrP27-30 (data not shown) confirming the mapping result in S. *cerevisiae* that laminin and PrP share the same binding site on LRP (amino acids 161-180).
For co-infection assays in insect cells including GST::LRP and prion proteins, the cellular form of the hamster prion protein PrP^{c} (PrP23-231) was synthesized and the soluble version (s) PrP27-30 of proteinase K resistant PrP27-30 (Caughey et al., 1991; Prusiner et al., 1981; 1983; 1984) was fused to the FLAG peptide. Both recombinant proteins were secreted into the culture medium, purified to homogeneity by anti-Flag-antibody affinity chromatography and recognized by a PrP specific antibody. Molecular weights determined by SDS-PAGE were 27 kDa and 17 kDa for rPrP^{c} and rPrP27-30, respectively, and correspond to rPrP^{c} (Weiss et al., 1995) and rPrP27-30 (Weiss et al., 1996) generated by thrombin cleavage from GST fusion proteins. In order to confirm the LRP/PrP interaction observed in S. *cerevisiae,* co-infection in Sf9 cells was performed. A specific interaction between the laminin receptor precursor and the prion protein PrP in insect cells confirms the PrP/LRP interaction observed in yeast.
To further confirm the PrP/LRP interaction in mammalian cells, FLAG::rPrP^{c} and authentic LRP were transiently expressed in Cos-7 cells. LRP revealed a molecular weight of 37 kDa and was recognized by a polyclonal anti-LRP antibody. rPrP^{c} fused to FLAG showed the expected molecular weight of 27 kDa and was also recognized by a polyclonal PrP specific antibody. Interaction between both proteins was proven in Cos-7 cells demonstrating that LRP interacts with PrP in a series of different eukaryotic cells including yeast, insect and Cos-7 cells.
The laminin receptor precursor family is highly conserved in a wide spectrum of eukaryotic cells (Wewer et al., 1986; Keppel et al., 1991; Garcia-Hernandez et al., 1994), was recently reported to be present in yeast (Demianova et al., 1996) and may be encoded by archaean genomes (Ouzounis et al., 1995). The receptor works as a receptor for Sindbis Virus on mammalian cells such as CHO or BHK (Wang et al., 1992) and for Venezuelan Encephalitis Virus from Mosquito Cells (Ludwig et al., 1996). For a functional involvement of the PrP^{c}/LRP interaction in the pathogenesis of TSEs or the biological function of PrP^{c} one would expect that the laminin receptor precursor is present in the human brain. Therefore, the presence of LRP-specific mRNA in multiple human tissues was tested. This analysis shows that LRP-mRNA is ubiquitously distributed in several tissues including heart and brain. The size of the laminin receptor precursor mRNA of approximately 1.4 kb is identical to that found in human, rat and mouse cells (Rao et al. 1989). In addition, it has been demonstrated that the 67 kDa LRP is present on neuronal cells (Kleinmann et al., 1988; Douville et al., 1988). Moreover, laminin itself has a drastic influence on the growth of neuronal cells. The LRP works as a receptor for the Sindbis-Virus and the VEE-virus on the surface of mammalian cells. The cellular prion protein PrP^{c} is also located on the surface of neuronal cells fixed by the glycosylphosphatidylinositol anchor (GPI; Stahl et al., 1987). Thus, a direct interaction of PrP^{c} and LRP is conceivable on the surface of neuronal cells of the brain. To further prove whether the laminin receptor precursor is overexpressed in Scrapie infected N₂a cells (Sc N₂a), crude lysates of N₂a and Sc N₂a cells with a LRP specific antibody were analyzed. LRP is present in crude lysates of N₂a cells but levels of LRP in crude lysates of Sc N₂a cells are twofold higher compared to N₂a cells suggesting that LRP expression is up-regulated in N₂a cells after Scrapie exposure. Recently, it has been reported that a soluble form of PrP27-30 was identified in human platelets (Perini et al., 1996). sPrP27-30 could work as a precursor for proteinase K resistant PrP27-30, a major product in Scrapie infected prion-preparations (Prusiner et al., 1981; 1983; 1984). The laminin receptor precursor has also been identified in human platelets (Tandon et al., 1991) which makes a PrP/LRP interaction also conceivable in platelets.

From the above results it can be concluded that LRP is required for the proliferation of PrP^{Sc}. A model of the function of LRP is discussed in the following section.

PrP^{c} is synthesized at the rER and secretes via the ER, the golgi and secretory vesicles passing through the plasma membrane to the cell surface. PrP^{c} is located on the surface of neuronal and other cells fixed by a glycosyl-phosphatidyl-inositol (GPI) anchor (Stahl et al., 1987). Treatment of the cell with phospholipase C (PIPLC) removes the diacylglycerol portion of the anchor releasing PrP^{c} from the cell surface (Lehmann and Harris, 1996). Mutants of PrP^{c}, however, remain bound to the membrane after PIPLC-treatment suggesting that PrP^{c} could interact with another unknown factor (Lehmann and Harris, 1996). PrP^{c} becomes internalized from the cell surface via clathrin coated pits (Shyng et al., 1994, 1995), caveolae or endocytosis (Taraboulos et al., 1995; Vey et al., 1996).

However, the location (secretory pathway/cell surface/endocytic pathway) where the conversion of PrP^{c} to PrP^{Sc} occurs remains unclear. PrP^{Sc} reaches the brain of TSE infected individuals via nerve cells or via the blood brain barrier. PrP^{Sc} could be transported from the periphery to the nerve cells by cells of the lymphatic system (such as B-lymphocytes which have been proven to be essential for prion propagation (Klein et al., 1997)) including the spleen, the peripheric nervous system (PNS) and the central nervous system (CNS) including the spinal cord. These cells might bind PrP^{Sc} via LRP. PrP^{Sc} could either bind to PrP^{c} on the cell surface in the absence or presence of an unknown receptor as suggested by Harris (Lehmann and Harris, 1996) such as the laminin receptor precursor. PrP^{c}/PrP^{Sc} binding could be dependent on the presence of the laminin receptor precursor. Membrane trafficking of PrP^{c} and PrP^{Sc}, either separately or complexed, can also be mediated by the laminin receptor precursor. The uptake of both PrP molecules can occur, either complexed or separately, by clathrin coated pits (Shyng et al., 1994, 1995), endocytosis or caveolae (Taraboulos et al., 1995; Vey et al., 1996). Heterodimeric complexes are formed in early endosomes, transport vehicles and endolysosomes. Lysosomes (Caughey et al., 1992; Taraboulos et al., 1992) are thought to contain predominantly PrP^{Sc} (Caughey et al., 1991). It is thought that PrP^{c} is still GPI anchored in endosomes, endolysosomes and lysosomes.

There is evidence that some early steps in the generation of PrP^{Sc} from mutant PrPs might take place in the ER or Golgi (Lehmann and Harris, 1996). Mutant PrP could be attached to the cell membrane in addition to the GPI-anchor via a transmembrane region or an additional receptor (Lehmann and Harris, 1996). LRP is most likely located on the cell surface since it was proven that LRP acts as a receptor for the Sindbis virus on mammalian cells such as BHK cells (Wang et al., 1992). Recently, it was shown that the Venezuelan Equine Encephalitis Virus (VEE) also uses LRP on the surface of mosquito cells. Thus LRP and PrP^{c} could share the same location on the surface of the cell.

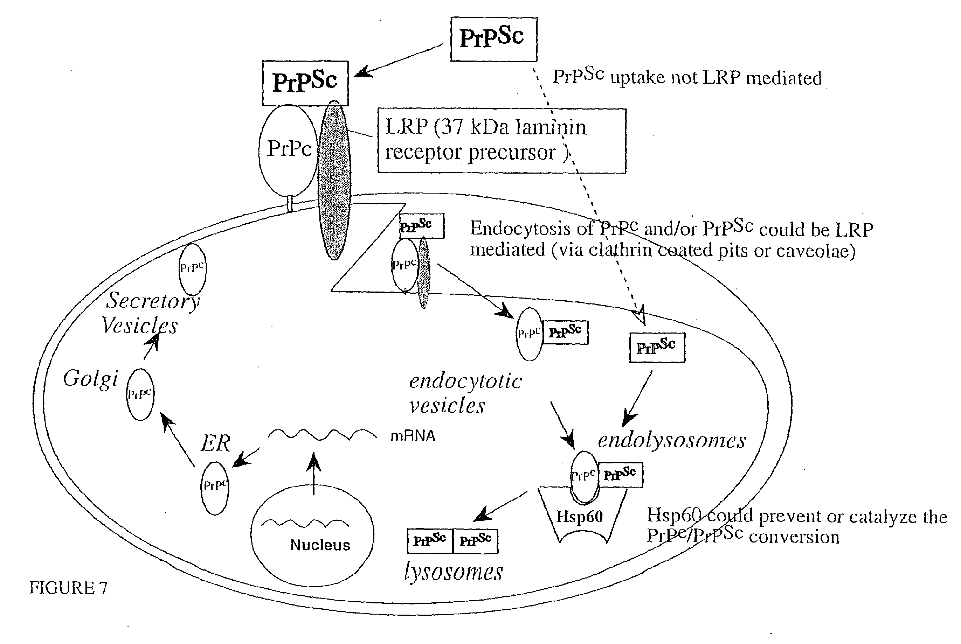

In the present invention it was observed, in addition, that LRP is overexpressed in Scrapie infected neuroblastoma cells. The binding of PrP^{Sc} to LRP could lead to the induction of LRP expression via a second messenger (maybe cAMP coupled) or an alternative mechanism. Alternatively, it is conceivable that PrP^{Sc} prevents LRP from degradation. The high concentration of LRP makes it likely that LRP is affiliated with PrP propagation by interacting with PrP^{c} or PrP^{Sc} or both (Fig. 7). Thus, by inhibiting said interaction using a soluble LRP or by trapping PrP^{Sc} diseases associated with the presence of PrP^{Sc} can be treated.

Accordingly, the present invention relates to a pharmaceutical composition comprising a soluble LRP, a mutein, fused protein, salt, functional derivative or fragment thereof, that can bind to PrP^{Sc}, PrP^{c} or fragments thereof. Said composition might be useful for the treatment of diseases associated with the presence of PrP^{Sc}.

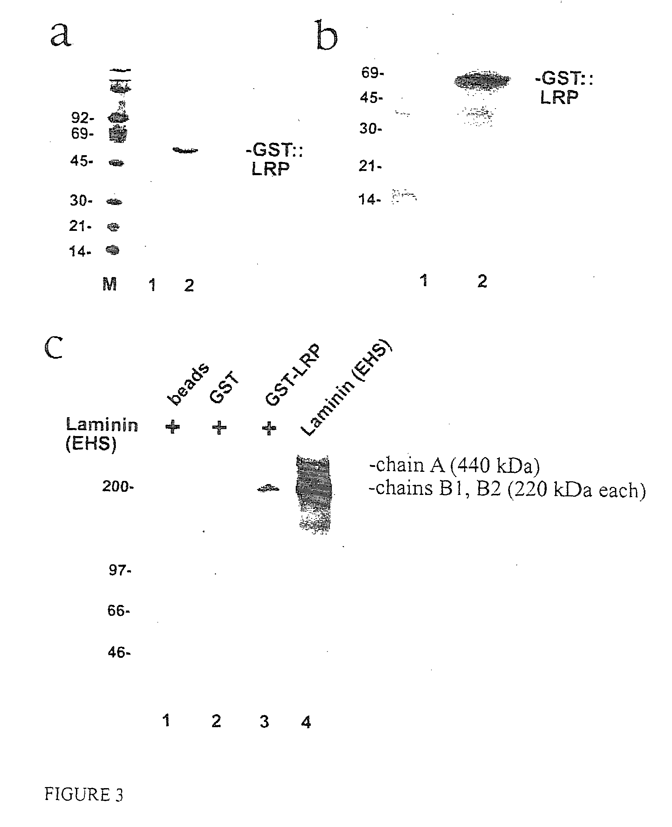

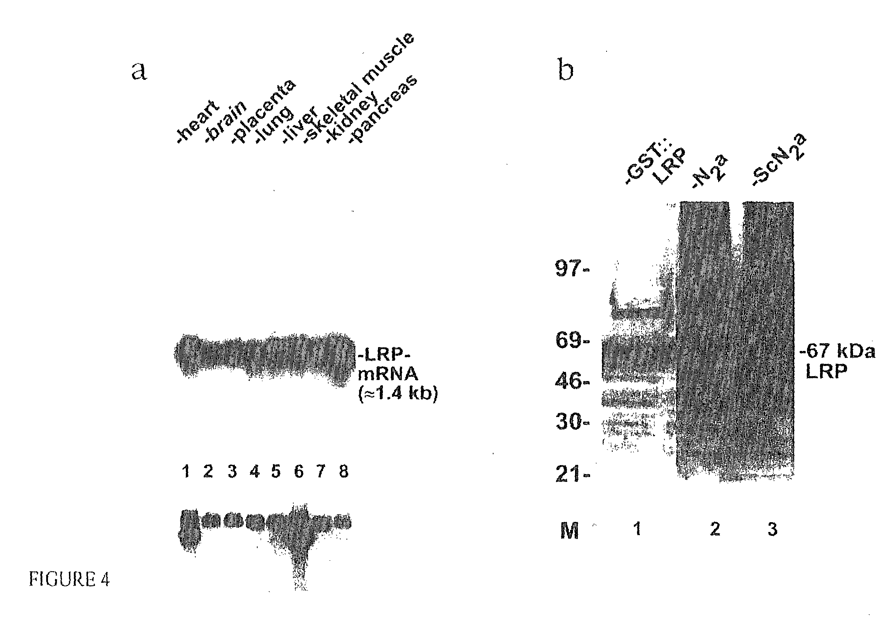

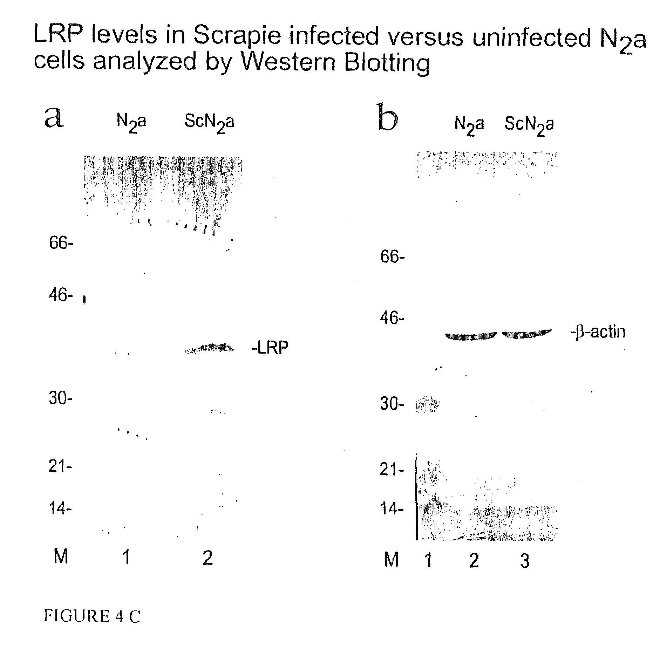

The soluble part of the LRP corresponds to the extracellular domain of LRP comprising the region from amino acid no. 101 to no. 295 of the amino acid sequence disclosed in Yow et al., 1988 (see Figures 3 and 4 and pages 6396 and 6397). It is, however, apparent for the person skilled in the art that for the preparation of the above pharmaceutical composition not only the soluble part of LRP with the amino acid sequence disclosed in Yow et al., 1988, can be used, but, in addition, a mutein of said soluble LRP, a fused protein, salt, functional derivative or fragment thereof which are still capable of binding PrP^{Sc} or PrP^{c}.

As used herein, the term "muteins" refers to analogs of the soluble LRP in which one or more of the amino acid residues of the soluble LRP described in Yow et al., 1988, are replaced by different amino acid residues or are deleted, or one or more amino acid residues are added without considerably changing the activity of the resulting product. These muteins are prepared by known synthesis and/or by site-directed mutagenesis techniques, or any other known technique suitable therefor.

The term "fused protein" refers to a polypeptide comprising a soluble LRP according to the invention, or a mutein thereof fused with another protein which has an extended residence time in body fluids. The soluble LRP may thus be fused to another protein, polypeptide or the like, e.g., an immunoglobulin or a fragment thereof.

The term "salts" herein refers to both salts of carboxyl groups and to acid addition salts of amino groups of a soluble LRP, muteins and fused proteins thereof. Salts of a carboxyl group may be formed by means known in the art and include inorganic salts, for example, sodium, calcium, ammonium, ferric or zinc salts, and the like, and salts with organic bases as those formed, for example, with amines, such as thiethanolamine, arginine or lysine, piperidine, procaine and the like. Acid addition salts include, for example, salts with mineral acids such as, for example, hydrochloric acid or sulfuric acid, and salts with organic acids such as, for example, acetic acid or oxalic acid.

"Functional derivatives" as used herein cover derivatives of a soluble LRP and its fused proteins and muteins, which may be prepared from the functional groups which occur as side chains on the residues or the N- or C-terminal groups, by means known in the art, and are included in the invention as long as they remain pharmaceutically acceptable, i.e. they do not destroy the activity of the protein and do not confer toxic properties on compositions containing it. These derivatives may, for example, include polyethylene glycol side-chains which may mask antigenic sites and extend the residence of a soluble LRP in body fluids. Other derivatives include aliphatic esters of the carboxyl groups, amides of the carboxyl groups by reaction with ammonia or with primary or secondary amines, N-acyl derivatives of free amino groups of the amino acid residues formed with acyl moieties (e.g. alkanoyl or carbocyclic aroyl groups) or O-acyl derivatives of free hydroxyl groups (for example that of seryl or threonyl residues) formed with acyl moieties.

As "fragment of the soluble LRP", its fused proteins and its muteins, the present invention covers any fragment of the polypeptide chain of the protein molecule alone or together with associated molecules or residues linked thereto, e.g. sugar or phosphate residues, or aggregates of the protein molecule or the sugar residues by themselves, provided said fraction has the same biological activity and/or pharmaceutical activity.

By using standard assays described in the literature, the person skilled in the art can easily check whether the above compounds are capable of binding PrP^{Sc}, PrP^{c} or fragments thereof.

The term "fragment of PrP^{Sc} or PrP^{c}" herein refers to any fragment of the polypeptide chain of the protein which can bind to the LRP.

The above pharmaceutical compositions may be prepared by any of the well known procedures in which the active ingredient, soluble LRP, is optionally admixed with pharmaceutically acceptable diluents, carriers or excipients. Actual dosages and modes of administration of such pharmaceutical compositions will be determined by the professional practitioners.

In addition, the present invention relates to a pharmaceutical composition comprising a compound that blocks the interaction of LRP and PrP^{Sc}, PrP^{c} or fragments thereof and, optionally, a suitable diluent, carrier and/or excipient. Such compounds can be identified, for example, by the screening system described below.

In a preferred embodiment, said compound is an antibody, preferably a monoclonal antibody, or a fragment thereof which binds to the surface epitope of LRP in such a way that binding of PrP^{Sc}, PrP^{c} or fragments thereof is no longer possible. Alternatively, the antibody binds to PrP^{Sc}, PrP^{c} or fragments thereof, thus blocking the LRP/PrP interaction.

Such antibodies or fragments thereof can be prepared according to standard procedures. Antibodies directed, for example, to the soluble part of LRP may be serum-derived (polyclonal) or monoclonal. For example, monoclonal antibodies are prepared using hybridoma technology by fusing antibody producing B cells from immunized animals with myeloma cells and selecting the resulting hybridoma cell line producing the desired antibody. Alternatively, synthetic peptides may be prepared using the amino acid sequence described in Yow et al., 1988. As a further alternative, DNA, such as a cDNA or a fragment thereof, may be cloned and expressed and the resulting polypeptide recovered and used as an immunogen. These antibodies are useful to inhibit the function of the LRP. Particularly useful are antibodies binding to the PrP binding domain of LRP (amino acids 161 to 180).

In a further preferred embodiment, said compounds which block the interaction of LRP and PrP^{Sc} or PrP^{c} comprise:
(1) peptides encoded by a peptide library,
(2) proteins encoded by a cDNA library, for example, derived from brain tissue or other appropriate organs,
(3) components of organic or anorganic nature synthesized chemically, including nucleic acids.

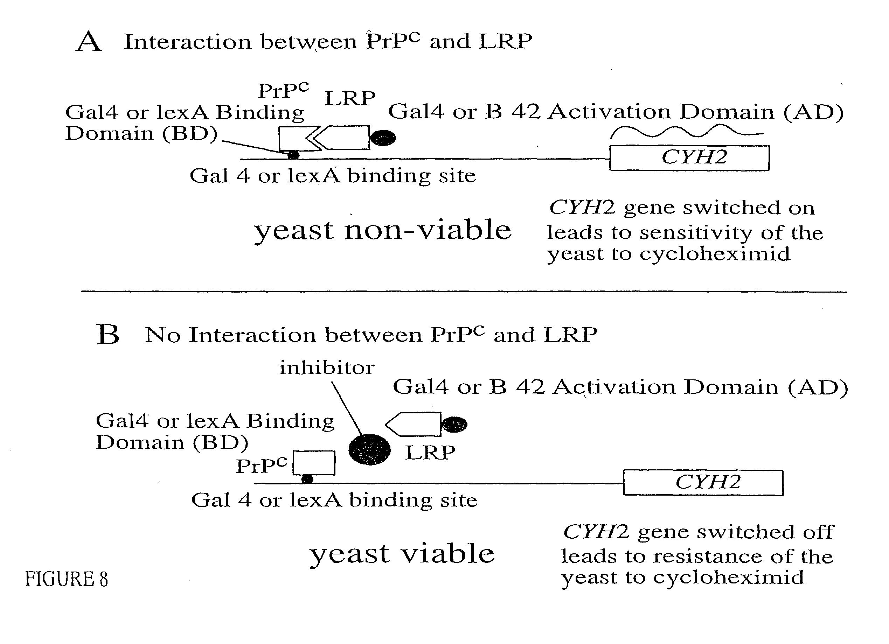

Among the above compounds those that inhibit the interaction between LRP and PrP^{Sc} or PrP^{c} can be identified, for example, by a reverse two-hybrid system. The reverse two-hybrid system (Fig. 8) modified from Leanna and Hannink, 1996, represents a straightforward method for the identification of compounds blocking the LRP/PrP interaction. The reverse Gal4/LexA two-hybrid system is a genetic selection scheme that selects against the association of PrP fused to the DNA binding domain Gal4/LexA and LRP fused to the activation domain of Gal4 or the acidic activation domain of B42. Association of Gal4::PrP/LexA/PrP and Gal4AD:LRP/B42:LRP results in the localization of the Gal4/B42 activation domain to the Gal1/LexA promoter and in the expression of CYH2. Survival of the CHX-resistant cyh2 yeast strain grown on plates containing CHX is dependent upon the lack of association of the PrP^{c} and LRP fusion proteins in the "bait" and "prey" position. The inhibitor can represent a compound added to the culture medium or a peptide expressed from a peptide cDNA library additionally transformed into the yeast strain. An additional selection marker (i.e. *His)* is necessary to select on the presence of the cDNA library. This system is described in more detail in Example 1, below.

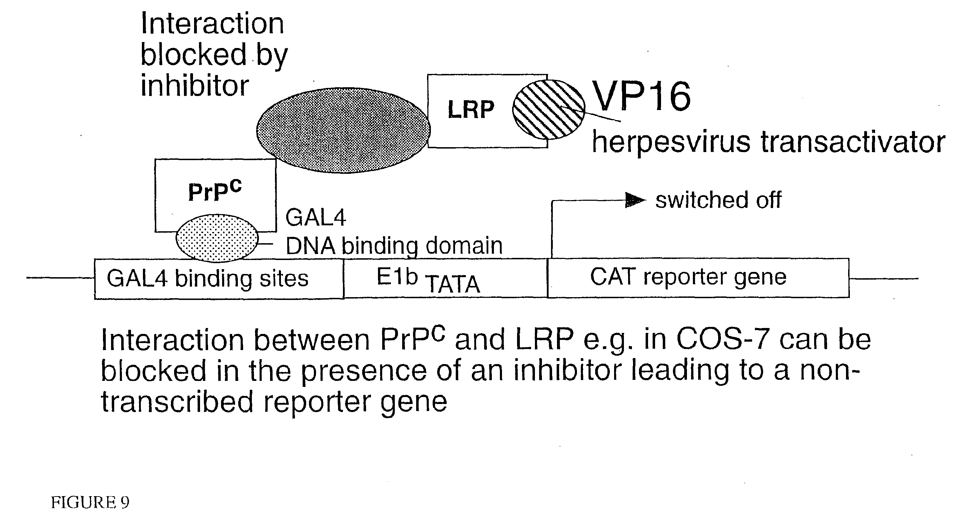

Alternatively, the mammalian two-hybrid system (supplied by CLONETECH Laboratories Inc., USA) can be used to screen for therapeutics interfering with the LRP/PrP interaction (Fig. 9). PrP can be fused to the DNA binding domain of Gal4 (bait plasmid). LRP can be fused to VP16, the herpesvirus transactivator in the prey position (prey plasmid). The interaction of bait and prey will lead to activation of transcription of the CAT reporter gene (supplied by a reporter plasmid) by RNA polymerase II from the minimal promoter of the adenovirus E1 b gene. Alternatively, luciferase can be used as a reporter gene enhancing the sensitivity of the assay.

Compounds can be screened using, for example, peptide libraries which can be co-transfected into mammalian cells using a selection marker such as neomycin (leading to viable cells in the presence of G 418). Compounds can easily be added to the culture medium. In contrast to the yeast system, mammalian cells are lacking a cell wall so that the uptake/intake of the compounds is much more efficient compared to yeast. In addition, mammalian cells represent the highest phylogenetic system with the highest degree in glycosylations, phosphorylations and other posttranslational modifications. The toxicity of a compound is assayed simultaneously in the mammalian system, whereas the toxicity of a component directed against the LRP/PrP interaction in the yeast two-hybrid system has to be re-assayed in the mammalian system for toxicity.

In addition, the yeast and the mammalian systems can be altered with respect to simplifying the screening assay. cDNA encoding LRP and PrP^{c} can be cloned into the bait position either in a co-directional or opposite direction leading to the expression of both proteins from a single plasmid. The cDNA library encoding for peptides or other presumable PrP/LRP interfering components can be cloned into the prey position so that no additional plasmid has to be transformed or transfected into the yeast or mammalian cells, respectively.

Accordingly, the present invention also relates to pharmaceutical compositions comprising a compound identified by the method described above.

The above pharmaceutical compositions might be useful for the treatment of diseases associated with the presence of PrP^{Sc}. Examples of such diseases are the transmissible spongiform encephalopathies, such as scrapie, bovine spongiform encephalopathy, Creutzfeldt-Jakob disease, Fatal Familial Insomnia, chronic wasting disease and feline spongiform encephalopathy (FSE).

Moreover, diseases associated with the presence of PrP^{Sc} can be treated by blocking the synthesis of LRP, e.g. by applying an antisense-RNA or a ribozyme which can bind/cleave the mRNA encoding LRP, thus inhibiting translation, and expression of LRP.

The application of suitable antisense RNAs or ribozymes, including the transformation of cells and the construction of expression vectors, can be carried out by methods well known to the person skilled in the art, e.g. by the techniques described in Example 9, below.

Thus, the present invention also relates to pharmaceutical compositions comprising an antisense-RNA or ribozyme which blocks the expression of the LRP.

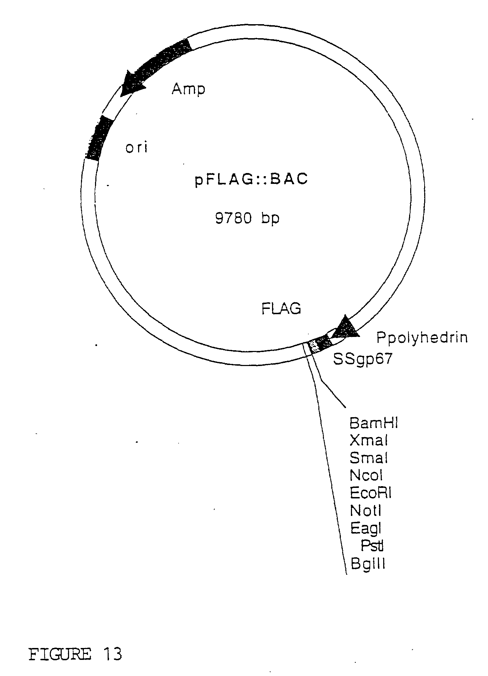

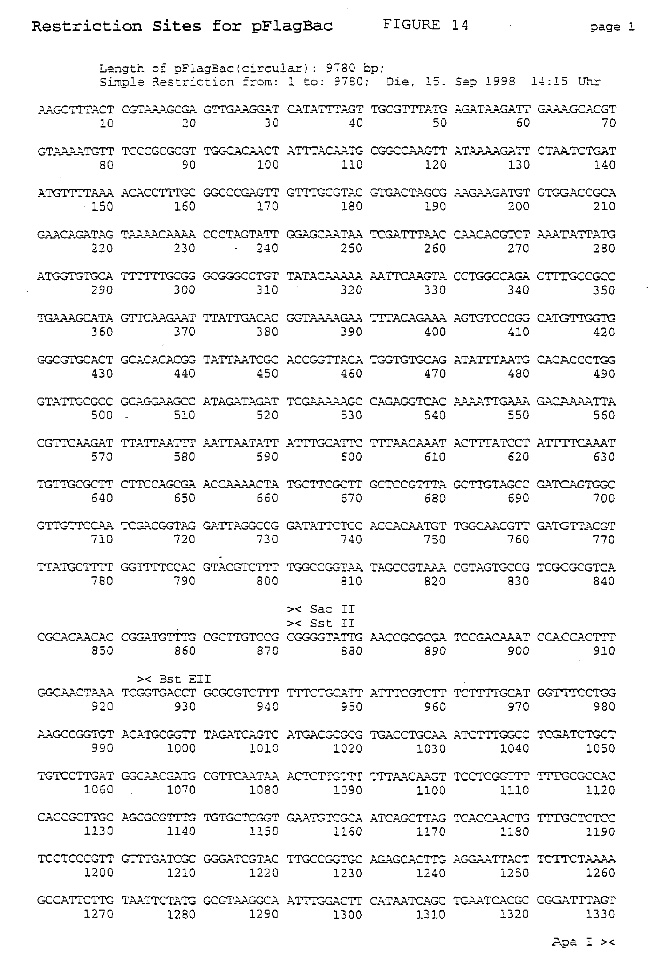

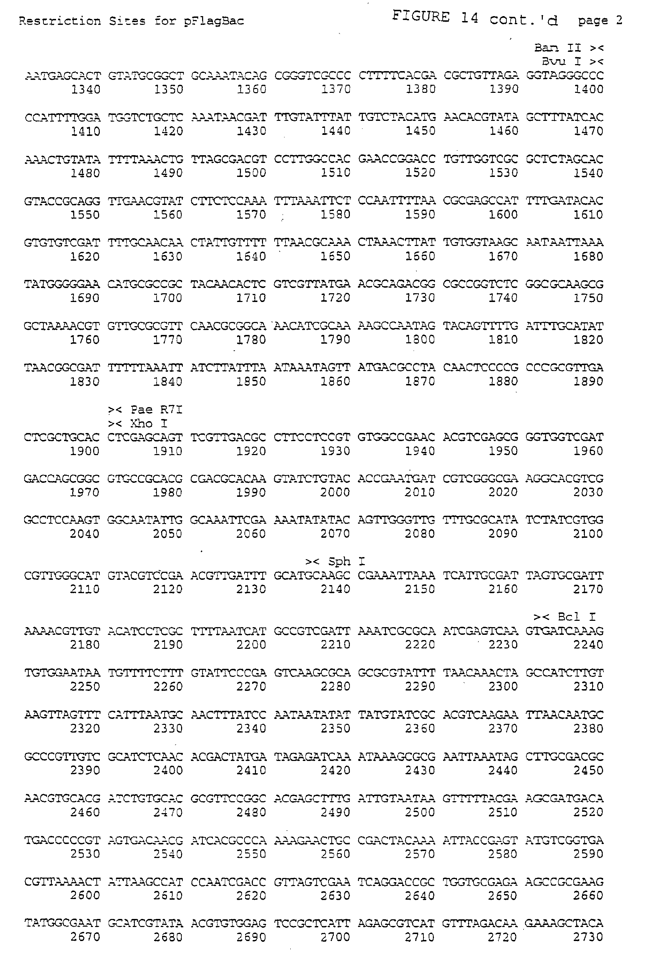

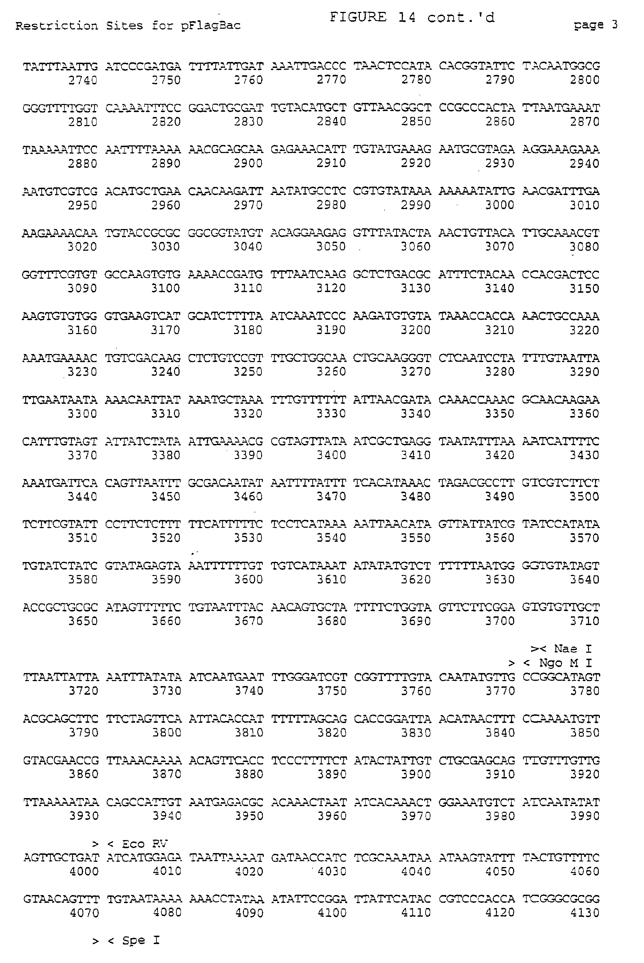

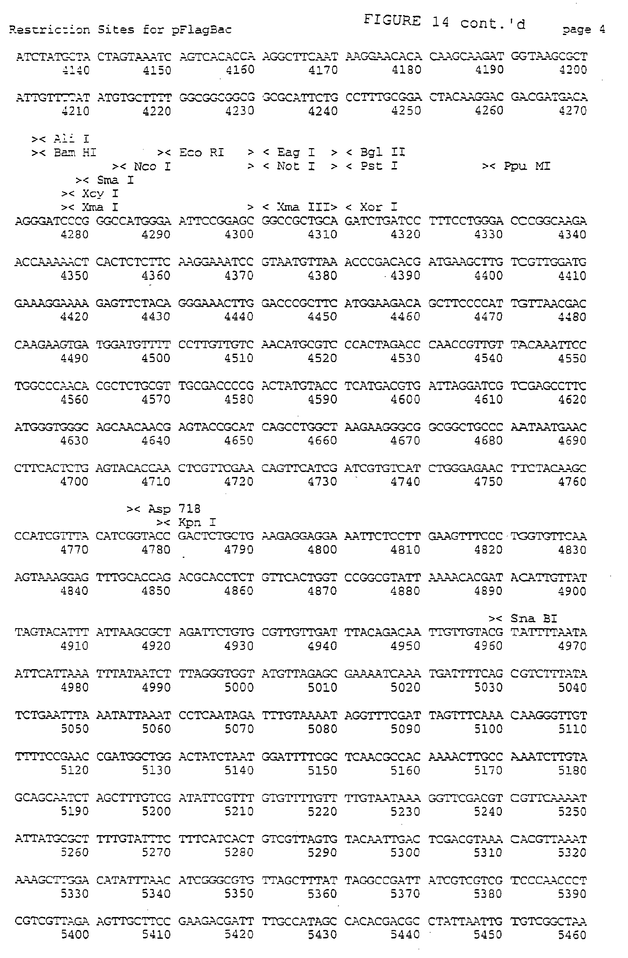

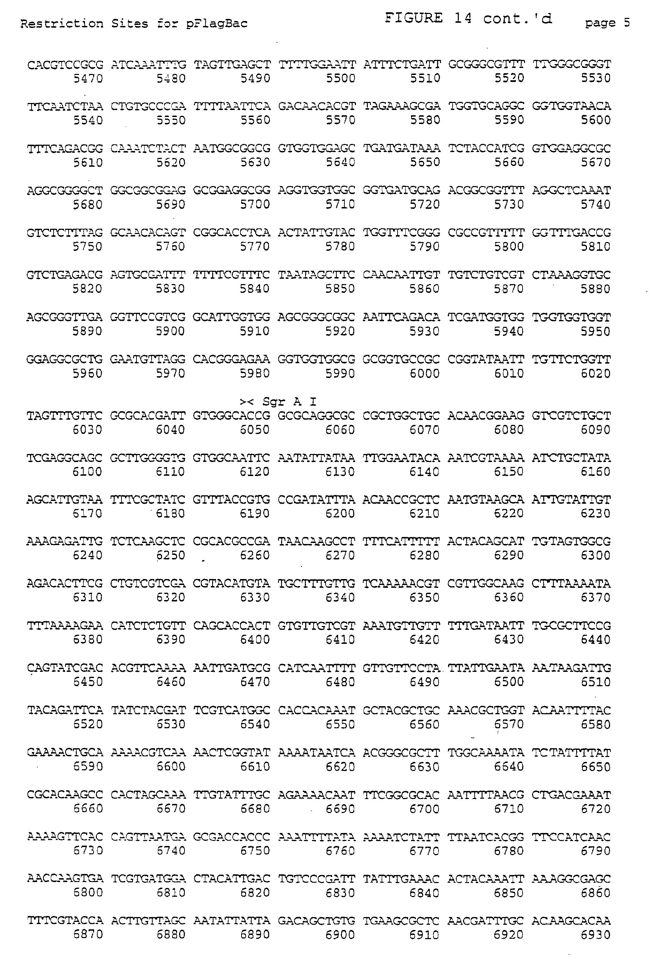

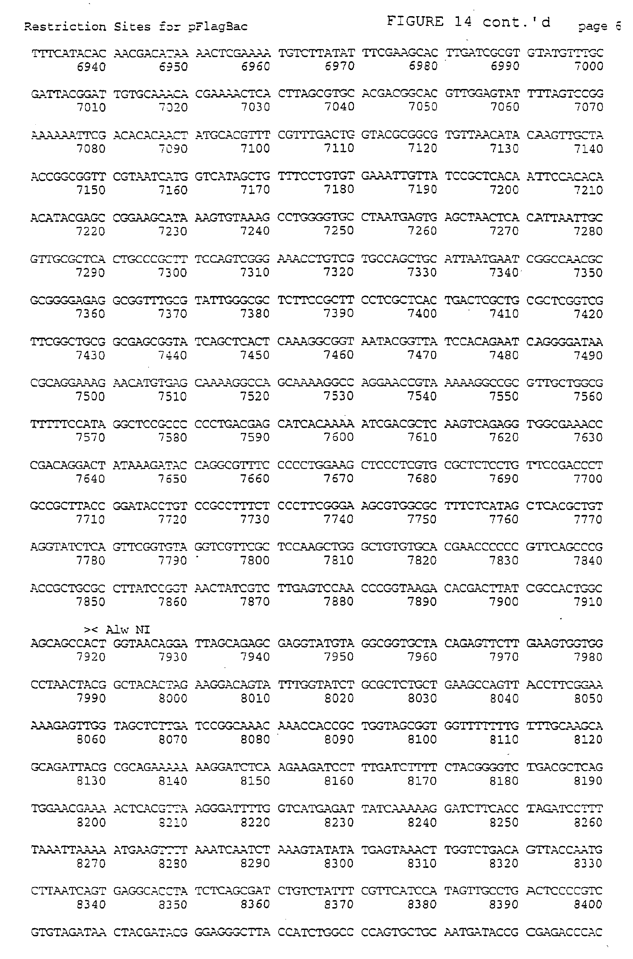

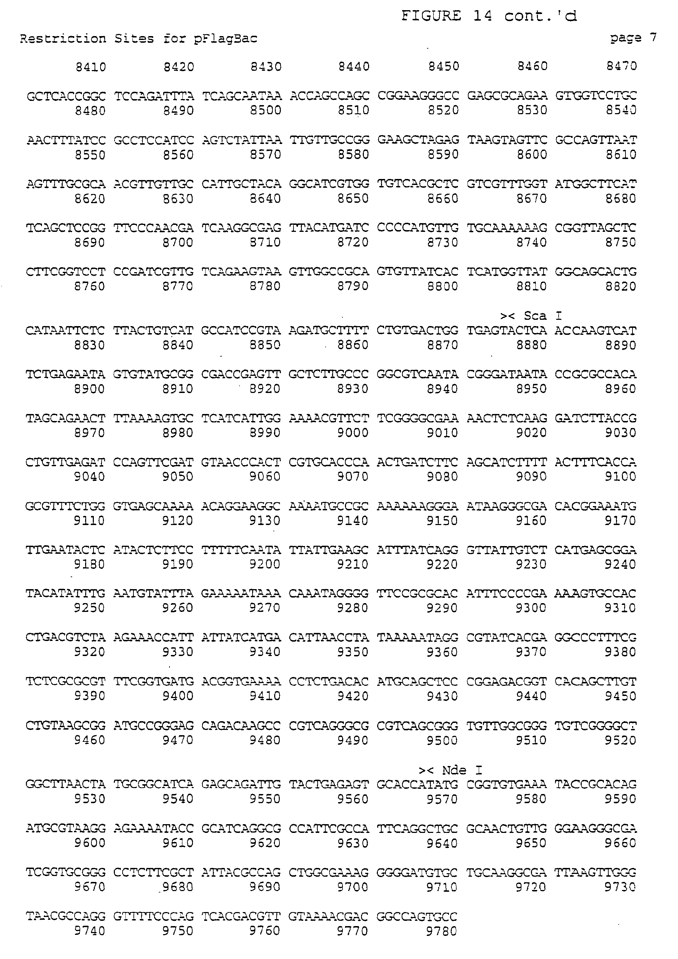

In addition, the present invention provides an expression vector for the recombinant production of LRP or any other kind of protein in the baculovirus system, pFLAG::BAC. The protein will be synthesized in the fusion with a FLAG-tag at the aminoterminal end of the protein. The FLAG-tag can be cleaved off employing the specific protease enterokinase. The recombinant protein will be secreted into the culture medium due to the presence of the signal sequence gp67. Proteins of any kind expressed in the fusion with FLAG in the baculovirus system can be phosphorylated and glycosylated, although it is known that the baculovirus system cannot form high-branched sugars. FLAG will enhance the solubility and stability of the fusion protein. This vector has the structure shown in Figure 13 and the nucleic acid sequence shown in Figure 14 or substantially said sequence.

The term "substantially", as used herein, refers to a nucleic acid sequence in which one or more nucleotides of the nucleic acid sequence shown in Figure 14 are replaced by different nucleotides or are deleted, or one or more nucleotides are added without considerably changing the biological properties of the vector as regards the capability (i) of replicating in the baculovirus system, (ii) of expressing the heterologous protein, (iii) of allowing the secretion of the protein in the culture medium and/or (iv) of providing the heterologous protein in a soluble and stable form. The nucleic acid sequence which has "substantially" the sequence shown in Figure 14 has at least 90%, preferably at least 95%, more preferably at least 98% and most preferably at least 99% homology to said sequence. Such a sequence is prepared by known synthesis and/or by site-directed mutagenesis techniques or any other known technique suitable therefor. The biological properties (i) and (iv) of a vector having such a sequence can be assayed by the skilled person using routine methods.

### Legends to the figures

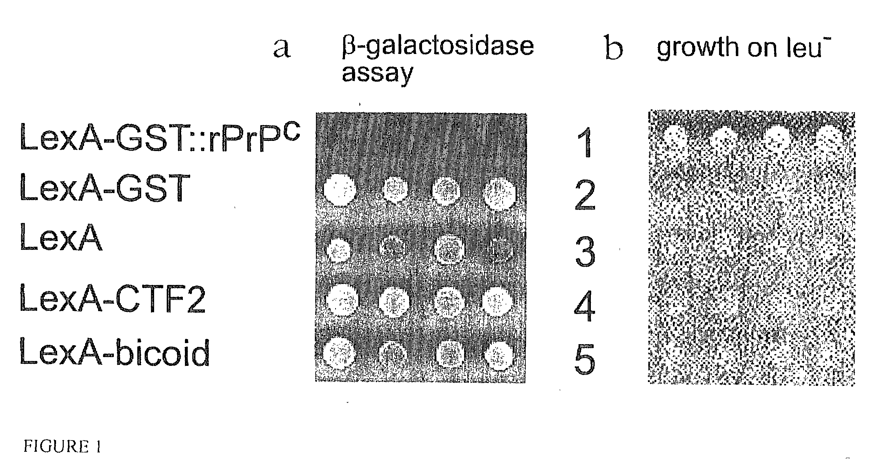

### Fig. 1: Identification of the PrP^{c}67 kDa LRP interaction in the yeast two-hybrid system in S. cerevisiae.

Yeast cells containing the reporter plasmid pSH18-34 were cotransformed with prey plasmid pJG4-5Δ43 encoding LRP (amino acids 44-295) encoding cDNA and the bait plasmids pSH2-1-GST::rPrP^{c} (row 1), pSH2-1-GST (row 2), pSH2-1 (row 3), pSH2-1-CTF-2 (row 4; Wendler et al., 1994) and pEG202-bicoid (row 5; Gyuris et al., 1993). Resuspension of each of the five transformants was carried out in TE and dotted onto galactose plates which were either supplemented with X-gal (a) or leucine deficient (b). Plates were incubated for 3 days at 30°C.

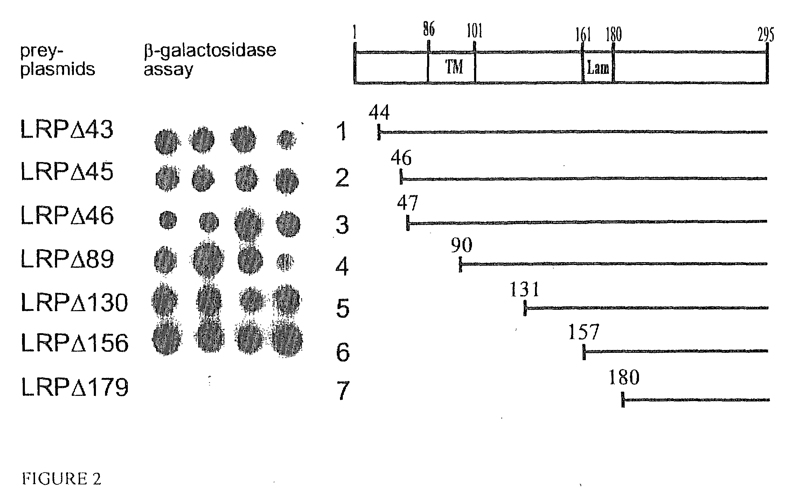

### Fig. 2: Mapping of the PrP/LRP interaction site in the yeast two-hybrid system

Yeast cells containing the reporter plasmid pSH18-34 were cotransformed with the bait plasmid pSH2-1-GST-PrP^{c} and prey plasmids pJG4-5Δ43 (row 1), pJG4-5Δ45 (row 2), pJG4-5Δ46 (row 3), pJG4-5Δ89 (row 4), pJG4-5Δ130 (row 5), pJG4-5Δ156 (row 6) and pJG4-5Δ179 (row 7) coding for amino terminal truncated versions of the laminin receptor precursor lacking 43, 45, 46, 89, 130, 156 amino acids (the latter contains 23 amino acids not homologous to the laminin receptor precursor) and 179 amino acids (lacking the laminin binding domain) at the amino terminus. Each of five transformants were resuspended in TE, dotted on X-Gal supplemented plates and incubated at 30°C for 3 days.

### Fig. 3: Analysis of recombinant LRP fused to GST in baculovirus infected insect cells (Sf9)

(a) Protein fractions from the supernatant (lane 1) and the cell pellet (lane 2) of 1.5 x 10⁷ Sf9 cells infected with AcSG2T-LRP were purified by glutathione sepharose chromatography and analyzed on a 12.5 % SDS-gel. (b) Western Blot analysis of (a) using a polyclonal LRP specific antibody. (c) Analysis of the interaction of recombinant LRP with Engelbreth-Holm-Swarm (EHS) laminin. 50 µl each of glutathione sepharose beads (lane 1), coupled with 37 pMol GST (lane 2), or 37 pMol GST::LRP (lane 3) were incubated with 17.5 pMol EHS laminin. Complexes were analyzed on 12.5 % in TCM buffer, reactions were carried out overnight at 4°C, beads subsequently washed four times with TCM and analyzed on SDS-PA-gels AgNO₃ stained. Two micrograms each of the individual marker proteins (rainbow marker RPN 756; lane M) and 3 µg of EHS-laminin (lane 4) were loaded.

### Fig. 4: (a) Northern Blot Analysis of multiple human tissues. and (b) and (c) Western Blot Analysis of crude lysates of N₂a/ScN₂a cells

Total mRNA isolated from human heart (lane 1), brain (lane 2), placenta (lane 3), lung (lane 4), liver (lane 5), skeletal muscle (lane 6), kidney (lane 7) and pancreas (lane 8) was immobilized on a nylon membrane and probed with a radiolabelled DNA fragment corresponding to 550 bp of the human 67 kDa high affinity laminin receptor precursor cDNA. (bottom) Total mRNA as described above was assayed with a radiolabelled β-actin probe.
(b) Western blot analysis of crude lysates from N₂a and ScN₂a cells employing an LRP specific antibody (anti-LBP (laminin-binding protein) antibody, provided by Dr. Starkey, Montana, USA, see below 8. Antibodies). 25 µl each of crude lysates of N₂a (lane 2) and ScN₂a (lane 3) cells and 500 ng of recombinant GST::LRP were analyzed on SDS-PAGE, blotted onto PVDF membrane and developed by a LRP specific antibody.
(c) Western blot analysis of crude Iysates from N2a and ScN₂a cells employing an LRP specific antibody prepared as described below (page 26; 8. Antibodies). 20 µl each of crude lysates from N2a (lane 2) and ScN2a (lane 3) cells were analyzed on a 12.5 % SDS-PAA gel, blotted onto a nitrocellulose membrane and developed with (a) a LRP specific antibody and (b) a monoclonal anti-β-actin antibody. Two micrograms of the individual marker proteins (rainbow marker RPN 756, Amersham; lane 1) were loaded.

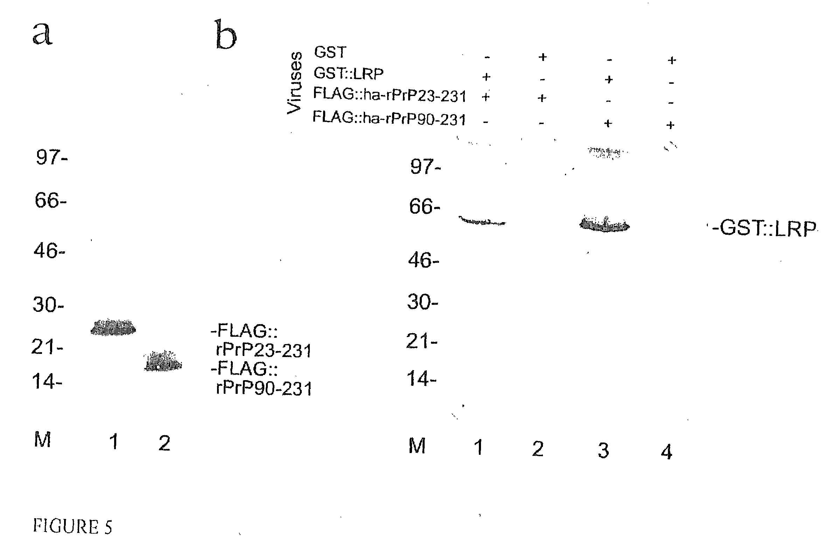

### Fig. 5: Analysis of PrP/LRP interactions by Co-infection assays in baculovirus infected insect cells (Sf9)

(a) Secretion of FLAG::rPrP23-231 (rPrP^{c}) and FLAG::rPrP90-231 from Baculovirus infected insect cells. Protein extracts from the supernatant of FLAG-BAC::rPrP23-231 and FLAG-BAC::rPrP90-231 (rPrP27-30) infected insect cells (1.5 x 10⁷) were purified by anti-FLAG-antibody affinity chromatography. 500 ng each of FLAG::rPrP23-231 (rPrP^{c}) (lane 1) and FLAG::rPrP90-231 (rPrP27-30) (lane 2) were and analyzed by western blotting developed by a polyclonal anti-PrP antibody. (b) Sf9 cells were co-infected with FLAG-BAC::rPrP23-231 and AcSG2T::LRP (lane 3), FLAG-BAC::rPrP23-231 and AcSG2T (lane 1), FLAG-BAC::rPrP90-231 (rPrP27-30) and AcSG2T (lane 4) and FLAG-BAC::rPrP90-231 (rPrP27-30) and AcSG2T (lane 2). GST and GST fusion proteins interacting with FLAG::rPrP23-231 and FLAG::rPrP90-231, respectively, have been isolated and purified by anti-FLAG-antibody affinity chromatography and analyzed by Western Blotting employing a polyclonal GST antibody.

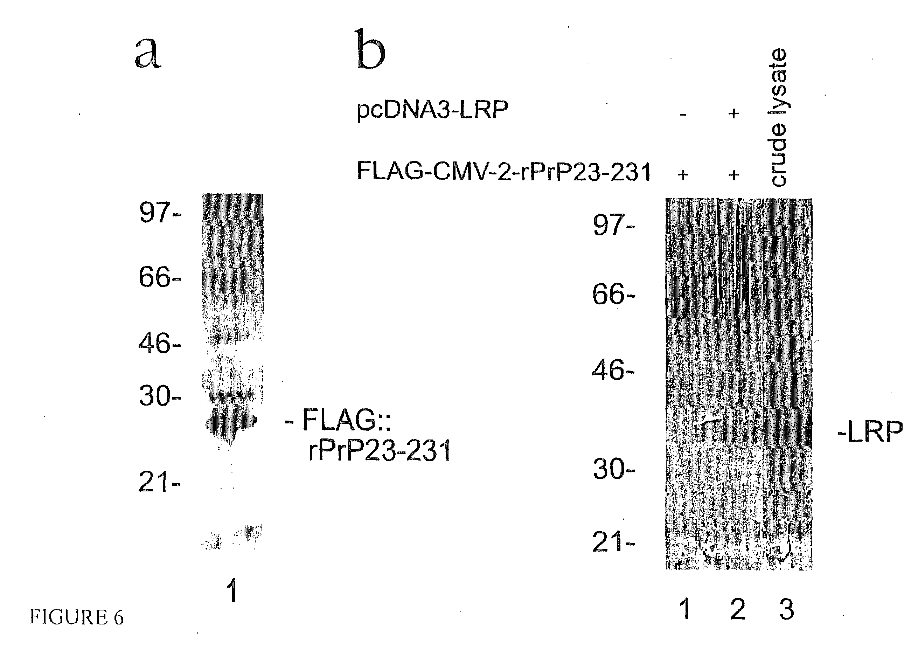

### Fig. 6: Analysis of PrP/LRP interactions by Co-transfection assays in Cos-7 cells

(a) Expression of FLAG::rPrP23-231 from hamster in Cos-7 cells. Lane 1: 500 ng of FLAG::rPrP23-231 purified from transiently transfected Cos-7 cells were analyzed by Western Blotting with an PrP specific antibody. (b) Co-transfection assay. Lane 2: Cos-7 cells were co-transfected with pFLAG-CMV-2::rPrP23-231 and pcDNA3::LRP. Cos-7 cells were transfected with pFLAG-CMV-2::rPrP23-231 (lane 1) or pcDNA3::LRP (lane 3). Crude lysate from pcDNA3::LRP transfected cells was analyzed by Western Blotting with a LRP specific antibody. LRP interacting with FLAG::rPrP23-231 was isolated and purified by anti-FLAG-antibody chromatography and analyzed by immunoblotting employing a polyclonal LRP antibody.

### Fig. 7: Model of the function of the LRP as the receptor for the prion proteins PrP^{c} and/or PrP^{Sc}

Endocytosis of PrP^{c} and eventually PrP^{Sc} via caveolae like domains or clathrin coated pits could be laminin receptor precursor (LRP) mediated. Conversion of PrP^{c} to PrP^{Sc} is thought to take place in the endolysosomes or lysosomes of the cell. Molecular chaperones are thought to be involved in that process. PrP replications can occur in brain cells such as neuroblastoma cells but also in other cells of the organism such as spleen and pancreatic cells. B-lymphocytes are thought to play a crucial role in transporting PrP^{Sc} to the nerve cell.

### Fig. 8: Schematic presentation of the reverse two-hybrid system to screen for components interfering with the LRP/PrP^{c} interaction

### Fig. 9: Schematic presentation of the mammalian two-hybrid system to screen for components interfering with the LRP/PrP^{c} interaction

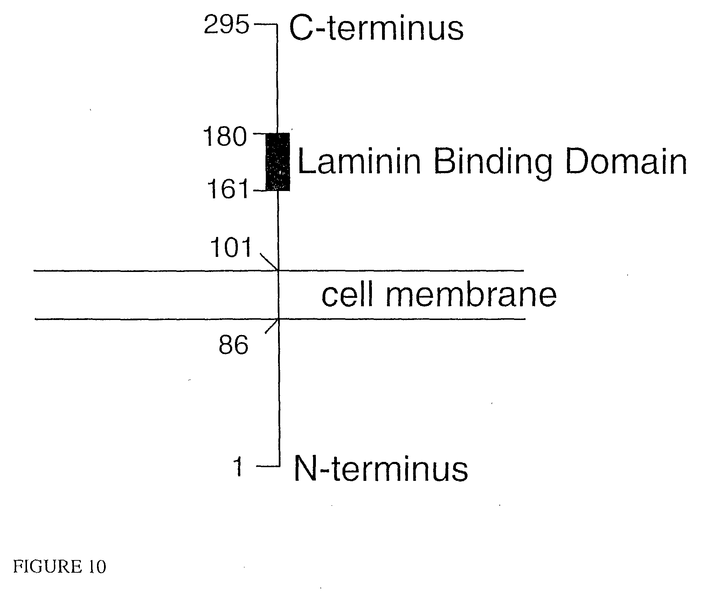

### Fig. 10: Localization of the 67 kDa high affinity laminin receptor precursor in the plasma membrane

The N-terminus of the receptor is thought to be located in the cytoplasm of the cell (Castronova et al., 1991). A short transmembrane region was also suggested between amino acid 88 and amino acid 101 of LRP (Mohane and Argos, 1986). The carboxy terminus of LRP is thought to be located extracellularly. A short laminin binding domain was identified between amino acid 161 and amino acid 180 of LRP corresponding to the PrP binding domain (Rieger et al., 1997, and Castronovo et al., 1991).

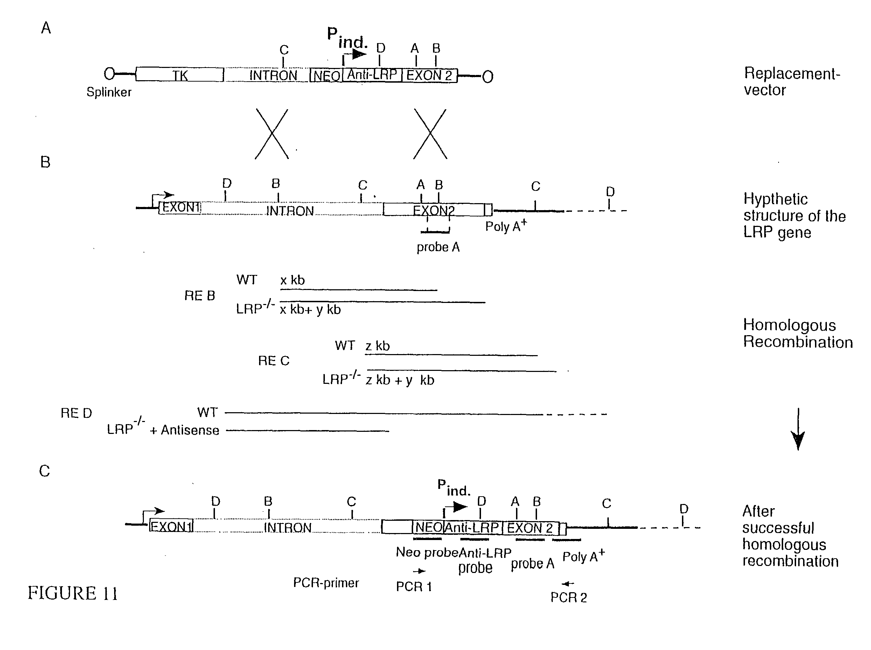

### Fig. 11: Targeted disruption of the LRP gene locus by homologous recombination and introduction of an Anti-LRP gene for antisense RNA production

Structure of the replacement vector (A), the hypothetic genomic structure of one LRP gene locus (B) and the mutated locus with the inserted antisense-LRP cassette (C). In the replacement vector parts of the exon will be replaced by the antisense-LRP cassette under the control of an inducible promoter (metallothionine promoter, tetracycline-responsive promoter) adjacent to the neomycin resistance cassette (NEO). The negative selection marker thymidine kinase (TK) will be added to one site of the homology region. Splinkers should be ligated to the linearized vector to prevent vector degradation by endonucleases. Shown are the probes for Southern blot analysis (probe A, Anti-LRP probe, Neoprobe). PCR primers to identify homologous recombined ES cell clones and predicted fragments after restriction enzyme (RE B, RE C, RE D) analysis are indicated. P_{ind} = inducible promoter.

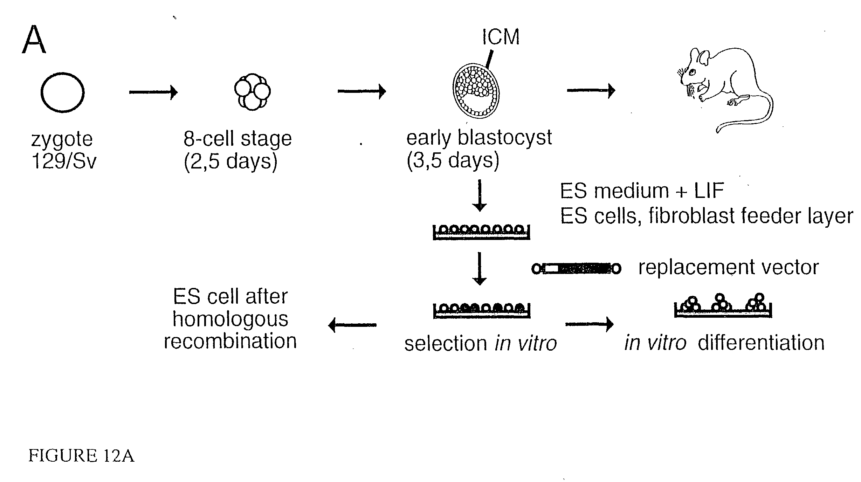

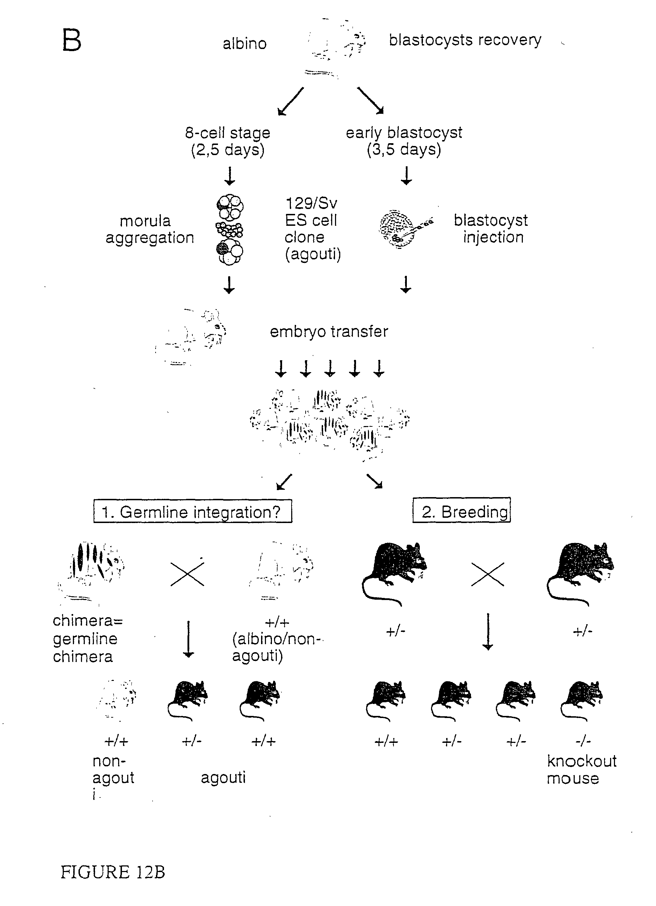

### Fig. 12: Generation of LRP knock-out mice

(A) ES-cells isolated from the inner cell mass of an early blastocyte are transfected with a replacement vector to produce ES-cell clones mutated on one allele of the LRP gene.
(B) Homologous recombined ES-cell clones were transferred into a mouse embryo via morula aggregation or blastocyst injection. Chimeras were assayed for germ line integration of the disrupted LRP gene by coat color and Southern blot analysis. Homozygous mice were obtained by inbreeding heterozygous mice. Abbreviations are: ICM, inner cell mass; ES, embryonic stem; LIF, leukaemia inhibitory factor.

### Fig. 13: Structure of the vector pFLAG:BAC

The map shows the position of the polyhedrin promoter, the signal sequence of gp67, the polylinker, the origin of replication and the gene conferring resistance to ampicillin.

### Fig. 14: Nucleic acid sequence of pFLAG:BAC with restriction sites

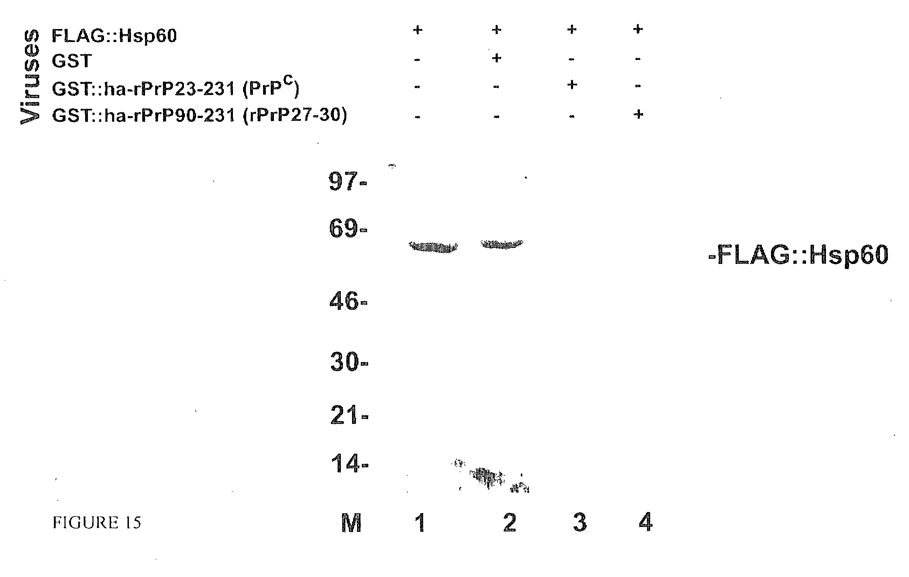

### Fig. 15: Co-expression of FLAG-tagged Hsp60 and GST-tagged prion proteins from Syrian golden hamster in the baculovirus system

Cell lysates were analyzed on 12.5% SDS gel. Western Blotting employing a monoclonal anti-FLAG M2 antibody shows decreased levels of FLAG::Hsp60 when co-expressed with GST::haPrP^{c}23-231 (lane 3) and GST::haPrP90-231 (lane 4) compared to FLAG::Hsp60 expression (lane 1) and co-expression of GST control (lane 2).

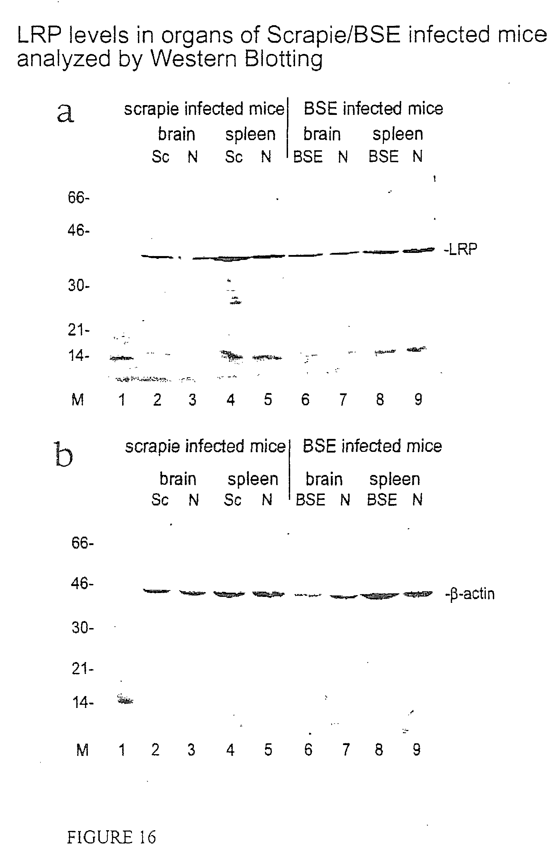

### Fig. 16: Western Blot analysis of brain and spleen homogenates from scrapie (strain C506M3) infected. BSE (strain 4PB1) infected and uninfected mice.

20 µl each of homogenates (corresponding to 0.5 mg of tissue) from brain of scrapie infected (lane 2), BSE infected (lane 6) and uninfected mice (lanes 3 and 7, respectively), 20 µl each of crude lysates from spleen of scrapie infected (lane 4), BSE infected (lane 8) and uninfected mice (lanes 5 and 9, respectively) were analyzed on 12.5 % PAA-gels and developed with (a) an LRP specific antibody and (b ) a monoclonal anti-β-actin antibody. Two micrograms of the individual marker proteins (rainbow marker RPN 756, Amersham; (a, b ) lanes 1) were loaded. Sc, BSE and N denotates Scrapie, BSE and not infected, respectively.

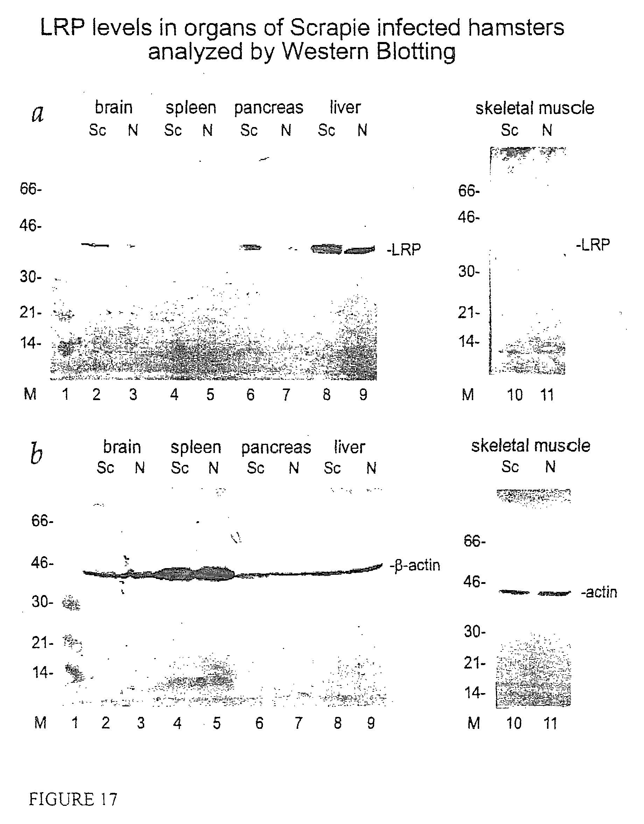

### Fig. 17: Western Blot analysis of homogenates of brain, spleen, pancreas, liver and skeletal muscle from scrapie (strain 263 K) infected and uninfected hamsters.

20 µl each of the homogenates (corresponding to 0.5 mg of tissue) of brain (lane 2), spleen (lane 4), pancreas (lane 6), liver (lane 8), skeletal muscle (lane 10) from Scrapie infected hamsters and 20 µl each of the homogenates of brain (lane 3), spleen (lane 5), pancreas (lane 7), liver (lane 9), skeletal muscle (lane 11) from uninfected hamsters were analyzed on 12.5 % SDS PAA-gels and developed with a LRP specific antibody (a), a monoclonal anti-B-actin antibody (b, left panel) and a monoclonal anti-actin antibody (b, right panel). Two micrograms of the individual marker proteins (rainbow marker RPN 756, Amersham; (a, b lanes 1) were loaded. Sc and N denotates Scrapie and not infected, respectively.

The results shown in Figures 16 and 17 are summarized in Table 1, below.

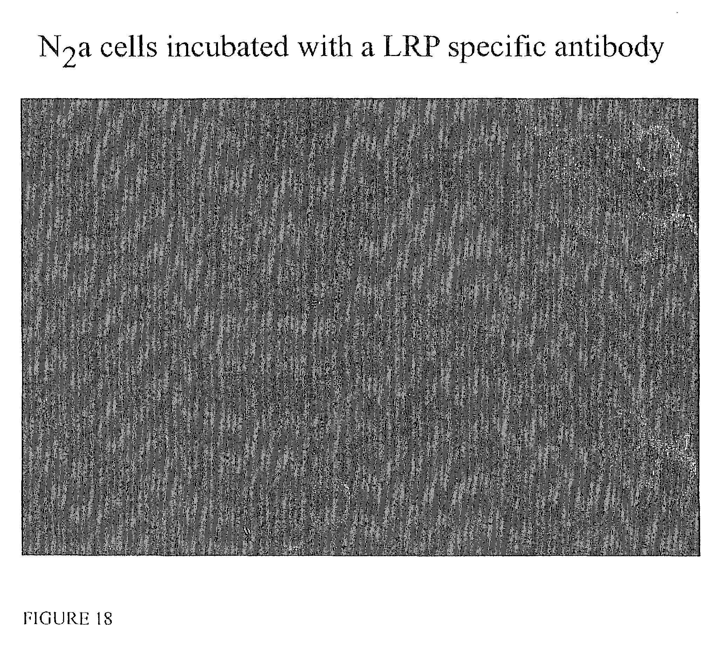

### Fig. 18: Localization of LRP on the surface of neuroblastoma cells (for details see Example 10)

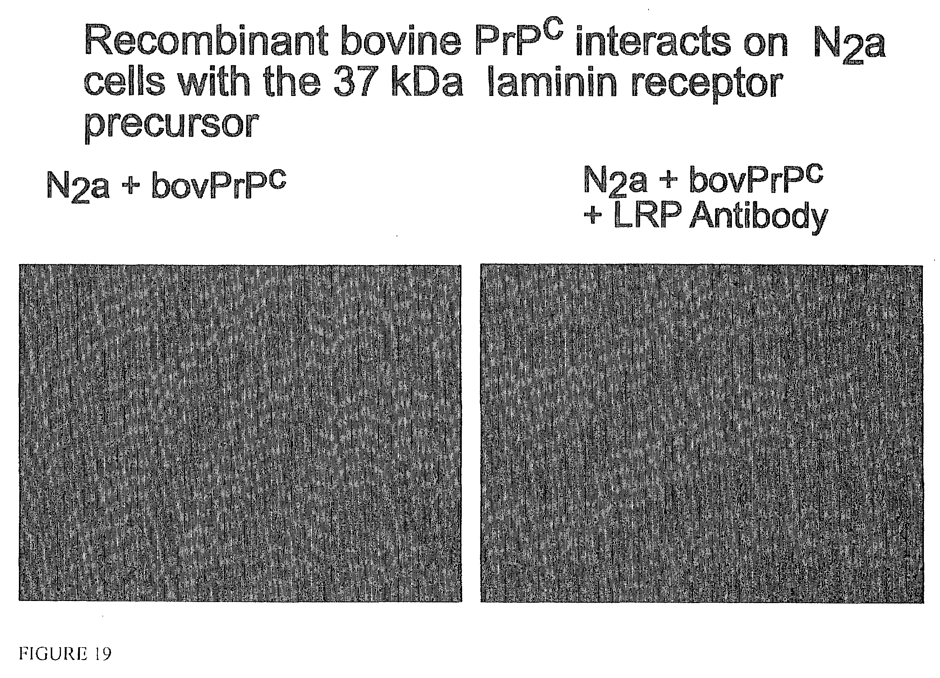

### Fig. 19: The Uptake of recombinant Prion Proteins from cattle by murine neuroblastoma cells (N₂a)

N₂a cells were incubated with bovine PrP fused to GST expressed in the baculovirus system. Cells were incubated with a PrP specific antibody (3B5) Magnification: 400 fold (A). A LRP specific antibody (Rieger et al., 1997) was preincubated before the recombinant bovine PrP was added to the cells followed by addition of a PrP specific antibody (3B5) Magnification 630: fold (B).

The invention will now be illustrated by the following examples. It should be noted that, unless otherwise indicated, the methods described in the examples are standard, well establishing methods, widely used in molecular biology. Accordingly, reference will be made to various publications in which the methods are fully detailed. Further, where details are provided concerning manufacturers, it is to be understood that the associated methods are according to the manufacturer's protocols.

General methods used in the Examples described below:
**1. Repression assay:**
   Each of the bait plasmids pSH-GST-PrP^{c}, pSH-2-1-CTF-2 and pEG202-bicoid were separately cotransformed with pJK101 (Gyuris et al., 1993), a reporter plasmid harboring LexA binding sites in the operator region of a lacZ reporter gene. In case of correct folding and transport in the nucleus the LexA fusion protein switches off the reporter gene resulting in white color of the colonies. *Control of bait protein expression:* Yeast cultures transformed with pSH-GST-PrP^{c} or pSH-GST were incubated for 2 days at 30° C. Cells were lysed in sample buffer and analyzed by SDS-PAGE and immunoblotting employing a hamster PrP-specific antibody.
**2. Plasmid constructions:**
   Cloning procedures were performed as described previously (Sambrook et al., 1989) unless otherwise stated.
   *(i) Construction of pAcSG2T::LRP*. The LRP (amino acid 44 to amino acid 295 of human 67 kDa high affinity laminin receptor precursor, accession no. IO2029) encoding cDNA was amplified by PCR from prey-plasmid pJG4-5 111.93 introducing a *Bam*HI and a 3' *Eco*RI-restriction site at the 5' and 3' end, respectively. The 768 bp fragment was cloned into baculovirus transfection vector pAcSG2T (Wang et al., 1995) via these two restriction sites resulting in pAcSG2T::LRP which was confirmed by didesoxysequencing.
   *(ii) Construction of pFLAG-BAC.* A DNA fragment encoding the carboxy terminus of the gp67 signal sequence (Whiteford et al., 1989) followed by the FLAG-tag (Asp-Tyr-Lys-Asp-Asp-Asp-Asp-Lys) flanked by a Spel (5') and a *Bam*HI (3') restriction site was PCR amplified from pAcGP67 B (Pharmingen) using oligodeoxyribonucleotide 1 (5' CTATGCTACTAGTAAATCAGTCAC 3') and oligodeoxyribonucleotide 2 (5' CCCATGGCCCGGGATCCCTTGTCATCGTCGTCCTTGTAGTCCGC AAAGGCAGAATGCGC 3'; the sequence encoding the FLAG-tag is underlined). The 139 bp DNA fragment was subcloned via Spel and *Bam*HI restriction sites into pAcGP67 B resulting in the baculovirus transfer vector pFLAG::BAC (9780 bp) which was proven by dideoxysequencing.
   *(iii) Construction of pFLAG-BAC::rPrP23-231 (rPrP*^{*c*}*)* The rPrP23-231 (rPrP^{c}) cassette (648 bp) was excised from pAcSG2T::PrP^{c}23-231 (Weiss et al., 1995) via *Bam*Hl and *Eco*Rl at the 5' and 3' end, respectively, subcloned into pFLAG-BAC resulting in pFLAG-BAC::rPrP23-231 (rPrP^{c}) (10406 bp) which was confirmed by dideoxysequencing.
   *(iv) Construction of pFLAG-BAC::rPrP90-231 (rPrP27-30).* cDNA encoding for rPrP90-231 (rPrP27-30) (447 bp) was restricted from pAcSG2T::rPrP27-30 (Weiss et al., 1996) with *Bam*Hl (5') and *Eco*Rl (3') and subcloned into pFLAG-BAC. The resulting transfer vector pFLAG-BAC::rPrP90-231 (rPrP27-30) (10205 bp) was confirmed by dideoxysequencing.
   *(v) Construction of pcDNA3::LRP.* The LRP (amino acid 44 to amino acid 295 of human 67 kDa high affinity receptor, accession no. 102029) encoding cDNA was amplified by PCR from expression plasmid pAcSG2T::LRP introducing a *Bam*HI and a *Eco*RI restriction site at the 5'and 3'end, respectively. The 844 bp fragment was cloned into mammalian expression vector pcDNA3 via these two restriction sites resulting in pcDNA3::LRP which was confirmed by didesoxysequencing.
   *(vi) Construction of pFLAG-CMV-2::rPrP23-231 (rPrP*^{*c*}*)*. A 97 bp DNA fragment encoding the N-terminal part of haPrP23-231 flanked by a *Hind*III (5') and Saul (3') restriction site was PCR amplified from pAcSG2T::rPrP23-231 (Weiss et al., 1995). A 558 bp DNA fragment encoding the carboxy terminus of haPrP23-231 was excised from pAcSG2T::rPrP23.231 (Weiss et al., 1995) via Saul (5') and *Eco*RI (3'). Both fragments were subcloned via *HindIII* and *Eco*RI into pFLAG-CMV-2 (Eastman Kodak Co.) resulting in the mammalian expression vector pFLAG-CMV-2::rPrP23-231 (5302 bp).
**3. Insect cell and Virus culture:**
   Cultures of *spodoptera frugiperda* (Sf9 cells) and infections by recombinant baculoviruses were performed as described previously (King and Possee, 1992; O'Reilly et al., 1992). Cotransfections employing 2 µg of pAcSG2T-LRP, pFLAG-BAC::rPrP23-231 (rPrP^{c}) and pFLAG-BAC::rPrP90-231 (rPrP27-30), respectively, and 0.5 µg of linearized baculovirus DNA were done as recently reported for GST::PrP^{c} and GST::rPrP27-30 (Weiss et al., 1995; 1996). Purified plaques were used to screen for the expression of GST::LRP, FLAG::rPrP23-231 (FLAG::rPrP^{c}) and FLAG::rPrP90-231 (FLAG::rPrP27-30) by SDS-PAGE and Western blotting. Recombinant virus stocks were amplified by picking plaques followed by reinfections of adherent Sf9 cultures in SF90011 in the absence of FCS. After several passages the recombinant virusstock reached 10⁷-10⁸ PFU/ml. Infections for the synthesis of recombinant protein were performed as described (Weiss et al., 1995; 1996). For protein synthesis as well as for virus stock amplification a multiplicity of infection (MOI) of 1 was used. For protein synthesis, medium containing FLAG::rPrP or cells containing GST::LRP were collected 72 h p.i. For the preparation of virus stocks, supernatants were harvested 4 to 7 days p.i.
**4. Co-infection studies in Sf9 cells:**
   FLAG tagged rPrP23-231 (rPrP^{c}) (Fig. 5 a, lane 1) and FLAG::rPrP90-231 (rPrP27-30) (Fig. 3 a, lane 2) secreted from baculovirus infected insect cells display molecular weights of 27 kDa and 17 kDa, respectively. GST::LRP can only be detected with a GST specific antibody upon co-infection with FLAG-tagged rPrP^{c} (Fig. 5 *b,* lane 1) and FLAG::rPrP27-30 (Fig. 3 *b,* lane 3). GST alone, however, is not detectable upon coinfection with FLAG-tagged rPrP^{c} (Fig. 3 b, lane 2) and FLAG::rPrP27-30 (Fig. 3 b, lane 4) or FLAG tagged controls, i.e. Src kinase p59hck (data not shown).
**5. Mammalian cell culture, transfection and co-transfection studies:**
   Cos-7 cells (ATCC # CRL 16512) were maintained as monolayer cultures in D10 medium (Dulbecco-modified Eagle media containing 10% fetal calf serum, 1% glutamine, 100 µg/ml penicillin, 100 µg/ml streptomycin as recommended by the manufacturer, Seromed). 1 x 10⁷ monolayered cells were either separately or simultaneously transiently transfected with 8 µg each of pcDNA3::LRP and pFLAG-CMV-2::rPrP23-231, respectively, using the lipofectamine transfection method according to the supplier's protocol (Gibco BRL). As a transfection control 1 x 10⁷ Cos-7 cells in monolayer culture were transiently transfected with pAAVRnIacZ (Chiorini et al., 1995) containing a nuclear-localized *LacZ* gene promoted by the Rous sarcoma virus long terminal repeat (RSV LTR) and flanked by an AAV ITR sequence. Cells expressing LRP and/or FLAG::rPrP^{c} were lysed 48 hr post transfection in 300 µl of PBS containing 0.1% Triton X-100. Lysates were either directly analyzed by SDS-PAGE and Western Blotting or in the case of an transfection assay with pFLAG-CMV-2::rPrP23-231 or co-transfection assay incubated overnight at 4°C with 100µl of a 1:1 slurry of anti-FLAG antibody-coupled agarose (Eastman Kodak) pre-equilibrated with PBS containing 0.1% Triton-X-100. The beads were subsequently washed in PBS and analyzed by SDS-PAGE and immunoblotting employing the anti-LRP antibody.
**6. Purification of GST::LRP:**
   For the purification of GST::LRP from AcSG2T::LRP infected Sf9 cells, cells were harvested 72-h-p.i. by centrifugation (700 x g for 10 min at 4°C), washed with PBSd (Weiss et al., 1995) and resuspended in PBSd supplemented with 0.1% Triton-X-100 at 4°C. Cells were lysed by centrifugation at 15000 x g for 10 min at 4°C and the protein from the supernatant immobilized by addition of 500 µl (per 1.5 x107 cells) of a 1:1 slurry of glutathione-Sepharose beads (delivered by Pharmacia and equilibrated in PBSd). After being shaken overnight at 4°C, beads were washed four times with 10 column volumes of PBSd and stored as a 1:1 slurry in PBSd at 4°C. GST::LRP was eluted from glutathione sepharose beads by competition with 18.3 mM reduced glutathione and shaking at 4°C overnight. Soluble protein was separated from the beads by centrifugation and analyzed by SDS-PAGE and immunoblotting. If desirable, glutathione can be removed by dialysis. The purification of recombinant GST::LRP from the culture medium was performed as described for the purification of FLAG tagged proteins.
**7. Purification of FLAG::rPrP23-231 (rPrP**^{**c**}**), FLAG::rPrP90-231 (rPrP27-30) and FLAG::Hsp60 from insect cells:**
   In order to purify FLAG::rPrP23-231 and FLAG::rPrP90-231 from either FLAG-BAC::rPrP23-231 or FLAG-BAC::rPrP90-231 infected Sf9 cells were harvested and lysed as described above. FLAG tagged protein from the supernatant of 1.5 x10⁷ cells was immobilized by the addition of 200 µl of a 1:1 slurry of Anti-FLAG M2 affinity gel over night at 4°C. Beads were washed four times with PBSd and directly analyzed by SDS-PAGE and immunoblotting (for protein analysis or the proof of protein-protein interactions). Soluble FLAG::rPrP^{c} and FLAG::rPrP27-30 was obtained by eluting the protein from the anti-FLAG affinity column by competition with 50 nMol FLAG-peptide (Kodak).
   The same approach was used to obtain FLAG::Hsp60. As shown in Figure 15 (lane 1), the protein is homogenous and migrates as a single band (61 kD) on SDS-PAGE.
**8. Antibodies:**
   For anti-GST-LRP antibody production 1 ml of a 1:1 slurry of GST::LRP immobilized on glutathione sepharose beads or 600 µl of soluble GST::LRP were added to 0.85% NaCl to a total volume of 2.6 ml. MPL + TDM + CWS Adjuvant System (RIBI adjuvant) was reconstituted with 2.0 ml of these solutions. After preparation of the preimmunserum two white albino rabbits [Charles River New Zealand; ZRL:kbl (nzw)br] each were 4 fold subcutan caudal immunized with 1 ml of immobilized and soluble GST::LRP. After 28 days the animals were boosted with immobilized and soluble GST::LRP as described above. After 14 more days the rabbits were immunized a third time as described. After 11 more days the animals were bled resulting in 200 ml of blood which was consequently coagulated for 1 h at 37°C and incubated over night at 4°C. The coagulated blood was centrifuged twice for 10 min each at 9000 rpm (in a GSA-rotor) and 10500 rpm at 4°C. 75 ml of antisera were stored at -20°C. Purification of the antibody was done by binding to a protein A-sepharose column (Pierce).
   Purified polyclonal anti-LBP antibody (laminin-binding protein corresponding to LRP) was kindly provided by Dr. J.R. Starkey, Montana. Polyclonal rabbit antibody directed against a peptide corresponding to a region comprising amino acids 95 to 110 of the Syrian Golden hamster prion protein was a generous gift of Dr. M. Groschup, BFAV Tübingen. Polyclonal GST antibody was delivered by Santa Cruz Biotech, USA.
   Anti-EHS-laminin antibody was purchased from Sigma.
**9. Animals:**
   C57BL/6 mice inoculated intracerebrally or intraperitoneally with experimental mouse scrapie (strain C506M3) or BSE (strain 4PB1) (Lasmézas et al., 1996) were sacrificed at terminal stage of the disease by cervical fracture. Different organs were immediately removed, frozen in liquid nitrogen and kept at -80°C until they were analyzed. The same procedure was applied to Syrian golden hamsters inoculated intracerebrally with scrapie strain 263K. Animals. C57BL/6 mice inoculated intracerebrally or intraperitoneally with experimental mouse scrapie (strain C506M3) or BSE (strain 4PB1)21 were sacrificed at terminal stage of the disease by cervical fracture. Different organs were immediately removed, frozen in liquid nitrogen and kept at -80°C until they were analyzed. The same procedure was applied to Syrian golden hamsters inoculated intracerebrally with scrapie strain 263K.
**10. Preparation of tissue homogenates from Scrapie and BSE infected and uninfected animals:**
   Brain (one whole hemisphere), spleen, pancreas, and pieces of liver and skeletal muscle were homogenized at 20% (w/v) in an isotonic glucose solution by means of a Ribolyser® (Hybaid). 280 µl of SDS-sample buffer were added to 40 µl of the homogenates, heated at 95° C for five minutes and centrifuged at 15000 RPM for 15 minutes. 20 µl (0.5 mg of total protein) were analyzed by western blotting.
**11. SDS PAGE and Immunoblotting:**
   Protein samples were analyzed on 12,5 % SDS Phastgels (Pharmacia) run on a Phastsystem as described previously (Heukeshoven et al., 1988) or Mighty Small gels according to the manufacturer's protocol (Hoefer). Following electrophoresis, gels were blotted onto polyvinyldifluorid membrane (PVDF) for 40 min. at 70° C in case of the phastgels or in a Semiphor Unit (Hoefer) in case of Mighty Small gels. Phastgels were stained automatically with silver in a development unit (Pharmacia) or with Coomassie Brilliant Blue as described. The blots were incubated with a polyclonal anti-LRP/anti-PrP antibody at a 1:100/1:800 dilution. The incubation steps were performed as described previously (Edenhofer et al. 1996). Antibody detection was performed by chemiluminescence (ECL system, Amersham).
**12. Laminin binding assay:**
   50 µl of glutathione sepharose beads either solely or coupled with GST (1 µg; 37 pMol) or GST::LRP (2,5 µg, 37 pMol) were equilibrated with TCM buffer containing 25 mM Tris.HCl pH 7.4 at 4°C, 5 mM MgCl2, 5 mM CaCl₂. After addition of Engelbreth-Holms-Swarm (EHS) laminin (Boehringer Mannheim; 3.5 µg; 17.5 pMol) in TCM buffer, reactions were carried out overnight at 4°C, beads subsequently washed four times with TCM and analyzed by Western Blotting employing an anti-laminin antibody (Sigma).
**13. Northern blot analysis:**
   A 550 bp DNA probe was generated by restriction of pJG-1.91 with *Asu*ll and *Eco*RI. The probe was ³²P-labeled using a random labeling kit (Gibco) and hybridized with a commercially available human multiple tissue Northern blot (Clontech). For controls a radiolabelled β-actin probe was hybridized. Hybridization was done with the ExpressHyb system (Clontech) at 68° C and the blot washed as recommended by the supplier. The blot was exposed for two days at -80° C with two intensifying screens.

### Example 1: Identification of LRP as a compound specifically binding to PrP by using the Yeast Two-Hybrid Screen

Construction of pSH2-1/pEG202-GST, pSH2-1/pEG202-PrP^{c}, pSH2-1/pEG202-GST-PrP^{c}, pSH2-1-PrP^{c}-GST was described in Edenhofer et al., 1996. As a bait protein we used the LexA (amino acids 1- 87) binding domain fused to GST-PrP^{c} screening a HeLa cDNA library fused to the acidic transactivation domain B42 in the pJG4-5 as described previously (Edenhofer et al., 1996). Potential positive EGY48 transformants were selected by growth on -Ura, -His, -Trp, -Leu in the presence of galactose due to the gal-promoter of the DNA-insert in pJG4-5. Positive clones were probed for β-galactosidase production by dotting them on galactose plates supplemented with 5-bromo-4-chloro-3-indolyl-β-D-galactopyranoside (X-Gal). The cDNAs of positive clones were recovered and transformed in *E*. *coli* KC8 as described (Edenhofer et al.,1996) and sequenced. For control experiments the plasmid pSH2-1, pSH2-1-GST, pSH2-1-GST::rPrP^{c}, pSH2-1-CTF-2 (Wendler et al., 1994) and pEG202-bicoid Gyuris et al., 1993) were retransformed in EGY48 and the transformants tested for β-galactosidase production and their Leu+-phenotype. At least five retransformants were dotted on corresponding plates and incubated for 3 days at 30°C.

Yeast strain EGY 48 was transformed with the pSH-GST-PrP^{c} construct coding for GST::PrP^{c} fused to LexA and tested for intrinsic activation as described previously (Edenhofer et al., 1996). Correct expression of the bait protein GST::PrP^{c} was confirmed by the repression activity of LexA. The control baits LexA-bicoid and LexA-Gal4 were also positive in the repression assay (data not shown). Expression of IexA-GST::PrP^{c} with a molecular weight of approx. 53 kDa was confirmed by western blotting developed with a polyclonal PrP specific antibody (data not shown). The PrP specific antibody did not recognize any proteins in the case of pSH-GST and pSH transformed yeast cells (data not shown). *S. cerevisiae* was cotransformed with pSH-GST-PrP^{c}, the reporter plasmid pSH18-34 and the prey plasmid pJG4-5, containing a HeLa-cDNA library controlled by a galactose-inducible promoter. A total of approximately 6 x 10⁵ transformants were pooled and an aliquot induced by adding galactose supplemented medium. Plating of the induced culture on Leu-deficient galactose plates resulted in approx. 2,000 colonies. 300 of them were screened for the β-galactosidase phenotype. The pJG4-5 plasmids of 55 clones were recovered and sequenced after retransformation in E. coli KC8 strain. Nine clones (corresponding to approximately 20 %) encoded N-terminal truncated forms of the 67 kDa High Affinity Laminin receptor precursor (LRP) with 43, 45, 46, 89, 130 and 156 amino acids lacking at the N-terminus. Specificity of the PrP-LRP interaction was demonstrated by several retransformation experiments. Cotransformation of pJG4-5 plasmid III.93 coding for the laminin receptor precursor (amino acids 44-295) and pSH-GST::PrP^{c} resulted in blue-colored colonies and complementation of leucin auxotrophy (Fig.1, row 1). Control plasmids pSH-GST encoding LexA-GST (Fig. 1, row 2) and pSH encoding LexA (Fig. 1, row 3) did not activate any of the reporter systems. The two 'false-baits' LexA-CTF2 (Fig. 1, row 4) and LexA-bicoid (Fig. 1, row 5) were also negative in both reporter assays (Fig.1, row 5) confirming the specificity of the PrP^{c} laminin receptor precursor interaction in S. *cerevisiae.*

### Example 2: Mapping of the PrP-LRP binding site in S. cerevisiae

Mapping of the PrP-LRP interaction site was investigated by several retransformation experiments with the plasmids pJG4-5Δ43, pJG4-5Δ45, pJG4-5Δ46, pJG4-5Δ489, pJG4-5Δ130, pJG4-5Δ156 pJG4-5Δ179 coding for amino terminal truncated versions of the laminin receptor precursor lacking 43, 45, 46, 89, 130, 156 amino acids (containing 23 amino acids not homologous to the laminin receptor precursor) and 179 amino acids (lacking the laminin binding domain) at the amino terminus. pJG4-5Δ43 to pJG4-5Δ156 were isolated from the two-hybrid screen described above. pJG4-5Δ179 was constructed by partial digestion of pJG-1.91 with *Ava*l followed by an *Eco*RI digestion. The resulting approx. 7 kb DNA fragment lacking the 420 bp *Eco*RI (5') - *Ava*l (3') DNA fragment was blunted and religated resulting in pJG4-5Δ179.

The N-terminally truncated versions of the receptor LRPΔ43 to LRPΔ156 resulted in blue colored colonies (Fig. 2, rows 1-6). Transformation of LRPΔ179, however, led to a negative X-gal phenotype (Fig. 2, row 7) demonstrating that laminin and PrP^{c} share the same binding site on LRP (amino acid 161 to amino acid 180).

### Example 3: Recombinant LRP synthesized in insect cells binds to Engelbreth-Holm-Swarm (EHS) laminin.

GST::LRP isolated from AcSG2T::LRP infected Sf9 cells (Fig.3 A, lane 2) migrates with a molecular weight of 67 KDa which was expected from the 27 kDa of GST (Weiss et al., 1995; 1996) plus 37 KDa observed from recombinant LRP expressed in highly aggressive cancer cells (Yow et al., 1988; Rao et al., 1989). No secretion of GST::LRP was observed (Fig.3 A, lane 1), despite the fact that the *autographica californica* gp67 signal sequence (Whitford et al., 1989) was constructed at the amino terminus of the fusion protein. A polyclonal LRP specific antibody specifically recognized the laminin receptor precursor fused to GST (Fig.3 B, lane 2) demonstrating that the recombinant LRP was immunologically active. To further prove the binding ability of the recombinant laminin receptor precursor to laminin immobilized GST::LRP was incubated in the presence of laminin from Engelbreth-Holm-Swarm (EHS) tumors. Laminin consists of two polypeptide chains B1 and B2 (each 220 kDa, Fig. 3 C, lane 4) linked via disulfide bonds to form together with polypeptide chain A (440 kDa, Fig. 4, lane 4) the characteristic asymmetric cross-structure. Binding of laminin can be detected in the presence of an anti-EHS-laminin antibody only with immobilized GST::LRP (Fig. 3 C, lane 3). No binding takes place in the presence of immobilized GST (Fig.3 C lane 2) or unloaded beads (Fig.3 C, lane 1).

### Example 4: LRP mRNA is present in several human organs including the brain

To identify mRNA encoding for the human LRP in various tissues a human multiple tissue Northern blot has been screened with a DNA probe complementary to a region encoding for the laminin receptor precursor. Hybridization of the labeled fragment to the human multiple tissue mRNA blot reveals that mRNA encoding for LRP is ubiquitous distributed in heart (Fig. 4A, lane 1), placenta (lane 3), lung (lane 4), liver (lane 5), skeletal muscle (lane 6), pancreas (lane 8) and the brain (lane 2). In contrast, LRP-mRNA level in kidney was very weak (lane 7).

### Example 5: LRP is overexpressed in ScN2a cells

Western Blot analysis of crude lysates with the LRP-specific antibody from Dr. Starkey (see 8., above) revealed that the 67 kDa LRP is present in the crude lysates of N2a cells (Fig. 4B, lane 2) but is overexpressed in Scrapie infected N2a cells (ScN2a) (Fig.4B, lane 3). The authentic LRP reveals the same molecular weight as recombinant GST::LRP (Fig. 4B, lane 1) from insect cells due to 27 kDa for PrP and 37 kDa for recombinant PrP. The presence of LRP in ScN2a cells favors the hypothesis discussed above that the receptor is involved in Scrapie propagation.

Moreover, LRP expression is tissue-dependent; see Table 1, above, and Figures 16 and 17.

To investigate the pathogenic relevance of the PrP/LRP interaction, LRP levels in Scrapie infected and uninfected murine neuroblastoma cells were analyzed with an LRP-specific antibody prepared as described in 8., above. Fig. 4c(a) demonstrates that the LRP level was twofold increased in Scrapie infected N₂a cells (lane 2) compared to uninfected N₂a cells (lane 1). The intensities of the protein bands were determined by optical scanning methods. The level of β-actin (42 kDa; control) remained stable in ScN₂a (Fig. 4c(b), lane 3) compared to N₂a cells (Fig. 4c(b), lane 2). The increased level of LRP in ScN₂a cells favors the hypothesis that the LRP is involved in scrapie pathogenesis.
Furthermore, LRP protein levels in brain and spleen of terminal C57BL/6 mice intracerebrally inoculated with either a scrapie (C506M3) or a BSE (4PB1) strain were investigated. The LRP level was twofold increased in brain and spleen of scrapie infected mice (Fig. 16a, lanes 2 and 4, respectively) compared to uninfected mice (lanes 3 and 5, respectively), whereas no significant alteration of the LRP amount was observed in either organ of BSE infected mice (Fig. 16a, lanes 6, 7, 8 and 9, respectively). Levels of β-actin (42 kDa) were not altered in scrapie /BSE infected animals (Fig. 16b). PrP^{Sc} accumulation is fourfold higher in the mouse scrapie model as compared to the mouse BSE model. Thus, the LRP concentration seems to correlate with the extent of PrP^{Sc} accumulation. This was further supported by the fact that no increased LRP concentrations were observed in brain and spleen of intraperitoneally inoculated scrapie mice which display only 50 % of the PrP^{Sc} accumulation observed in intracerebrally inoculated mice.
In order to confirm the direct correlation between LRP levels and PrP^{Sc} accumulation, we investigated the amounts of LRP in different organs of syrian hamsters inoculated with the 263K scrapie strain, which is a model with high levels of intracerebral PrP^{Sc} accumulation. The LRP level was fivefold increased in the brain (Fig. 17a, lane 2) and fourfold raised in the pancreas (lane 6) of scrapie infected hamsters compared to uninfected control hamsters (lanes 3 and 7, respectively). LRP is weakly expressed in the spleen (lane 5) and its level only marginally (1.5 fold) increased in this organ in scrapie infected hamsters (lane 4). These data fit with the high neuroinvasiveness of the 263K strain where PrP^{Sc} and infectivity are extremely high in the brain and, in contrast, low in the spleen. The pancreas exhibits PrP^{Sc} and pathological changes particularly located in the islets of Langerhans, although its role in the pathogenesis of prion diseases is poorly understood.
In contrast, LRP expression failed in skeletal muscle (lanes 10 and 11) and was unaltered in the liver of scrapie infected hamsters (lane 8) compared to uninfected hamsters (lane 9). β-actin or actin (42 kDa) levels, respectively, were unaltered in brain, spleen, pancreas, liver and skeletal muscle of scrapie infected compared to uninfected hamsters (Fig. 17b).
In summary, LRP levels are increased only in those organs that exhibit infectivity and PrP^{Sc} accumulation such as brain, spleen and pancreas (see Table 1, below).

**Table 1**

| Increase of LRP levels in Scrapie/BSE infected versus uninfected cells and tissues* | | | | | |
|---|---|---|---|---|---|
| MOUSE | brain | spleen | ScN₂a/ N₂a cells | | |
| Scrapie infected | 2 fold | 2 fold | 2 fold | | |
| BSE infected | 1.1 fold | unchanged | | | |
| | | | | | |
| HAMSTER | brain | spleen | pancreas | liver | skeletal muscle |
| Scrapie infected | 5 fold | 1.5 fold | 4 fold | unchanged | not expressed |

| | | | | | |
|---|---|---|---|---|---|
| *values have been calculated by optical scanning methods. | | | | | |

### Example 6: rPrP23-231 (rPrP^{c}) and rPrP90-231 (rPrP27-30) interact with LRP in insect cells co-infected with recombinant baculoviruses

FLAG tagged PrP^{c} (Fig. 5A, lane 1) and FLAG::rPrP90-231 (Fig. 5A, lane 2) secreted from Baculovirus infected insect cells revealed molecular weights of 27 kDa and 17 kDa, respectively. Co-infection assays demonstrate that GST::LRP can only be detected by co-infecting AcSG2T::LRP with FLAG-BAC::rPrP23-231 (Fig.5 B, lane 3) or FLAG::BAC::rPrP90-231 (Fig.5 B, lane 1). No interaction takes place when AcSG2T expressing GST was co-infected with FLAG-BAC::rPrP23-231 (Fig.5B, lane 2) or FLAG::BAC::rPrP90-231 (Fig.5B, lane 4). As an additional control GST::LRP failed to interact with Src kinase p59*hck* (Danhauser-Riedl et al., 1996) fused to GST (data not shown) demonstrating the specific interaction of rPrP23-231 and rPrP90-231 with LRP in insect cells.

### Example 7: rPrP23-231 (rPrP^{c}) interact with authentic LRP in Cos-7 cells

To further confirm the LRP/PrP interaction in mammalian cells, we co-transfected Cos-7 cells with plasmids encoding for authentic LRP and PrP^{c}. Recombinant LRP recognized by a LRP specific antibody revealed a molecular weight of 37 kDa (Fig. 6 B, lane 3) as expected from the gene product expressed from LRP-cDNA (Yow et al., 1988). FLAG::haPrP23-231 was synthesized as a 27 kDa protein (Fig. 6A, lane 1) and recognized by a PrP specific antibody. Co-transfection assays with LRP and rPrP23-231 proved the presence of LRP only after simultaneous expression of both proteins (Fig. 6 B, lane 2). In the absence of pcDNA3-LRP no interaction takes place (lane 1). In summary the interaction of the 67 kDa laminin receptor precursor with prion proteins was demonstrated in the yeast, in insect and mammalian cells.

### Example 8: LRP knock-out mice are resistant to PrP^{Sc} infection

The function of LRP as a receptor for PrP^{Sc} can be assayed by employing LRP knock-out mice. Because LRP belongs to a multicopy gene family (Bignon et al., 1991; Douville and Carbonetto, 1992; Fernandez et al., 1991), it is impossible to use the classical knock-out strategy recently described for the construction of a *bir*1-knock-out mouse (Förster et al., 1996). Thus, it is necessary to switch off additional loci. This can be done by connecting the classical knock-out approach with ah antisense strategy. This procedure leads to mice with one disrupted LRP gene. The mRNA of further active LRP genes can be inactivated by the induced antisense mRNA.
The first step in the generation of a LRP knock-out mouse is the screening of one homologue of the LRP gene in a genomic mouse library (129 SV) cloned in a λ-Fixll™ vector. Employing an LRP specific DNA probe leads to the identification of the genomic structure of the LRP gene, which is subcloned, mapped and sequenced. The second step includes the construction of a replacement vector (Fig. 11). Parts of the LRP gene are disrupted and replaced by the gene encoding neomycin resistance for positive selection (Fig. 11) adjacent to an anti-LRP cassette containing the antisense LRP sequence under the control of an inducible promoter such as the metallothionine promoter or tetracycline-responsive promoter (Gossen and Bujard, 1992). The thymidine kinase gene flanking the homology regions on either side of the LRP gene enables negative selection. The replacement vector is transferred to murine embryonic stem cells (ES) via electroporation (Fig. 12A). The presence of G 418 and gancyclovir leads to clones potentially harboring the homologous recombination event. Screening of positive selected clones via PCR and Southern blot analysis allows the identification of clones mutated in one LRP gene on one allele (Fig. 11, 12A).

Homologous recombined ES cell clones are transferred into embryonic morulas or blastocytes by morula aggregation and blastocyst injection. Chimeras are identified by coat color and tested for germ line integration by Agouti-coat color (to identify the integration of the homologous recombined embryonic stem cells) and Southern blotting. Heterozygous mice are intercrossed to produce homozygous LRP^{-/-} mice with one disrupted LRP gene. In order to inactivate the additional LRP genes, the metallothionine or tetracycline-responsive promoter (Gossen and Bujard, 1992) is activated by feeding the mice with Zinc or tetracycline, respectively. The antisense LRP mRNA binds additional generated LRP mRNA. This approach can be done during different developmental stages and can be proven by Northern blot analysis, Western blotting and FACS analysis.

### Characterization of the phenotype of the LRP^{-/-}mice

The phenotype of the LRP^{-/-} mice can be characterized including behaviours, histological examination of the brain and other organs and neurological investigations.

### Phenotypic characterization

The mice are monitored over their whole lifespan for the occurrence of clinical abnormalities including neurological signs. In addition, to evaluate the motor coordination of the mice objectively, their preformance is tested on a rotorod. With respect to the behavioural characterization, the learning ability of the LRP^{-/-} mice can be assessed and compared with WT mice possessing the same genetic background, by means of appropriate tests (e.g. swimming navigation and Y-maze discrimination test).
Anatomical investigations are made to detect gross abnormalities of the brain and other organs; histological examinations are peformed using classical techniques and haematoxylin-eosin staining.
The crucial experiment, however, is the innoculation of the LRP^{-/-} mice with PrP^{Sc-} enriched infectious fractions from mouse and cattle.
PrP^{Sc} detection procedure (Lasmezas et al., 1997). Mice infected with PrP^{Sc} are sacrificed at the pre-mortem stage by cervical fracture, and brains are immediately removed. One hemisphere (including the cerebellum) is frozen in liquid nitrogen, and stored at -80°C for PrP analysis. The other hemisphere is fixed for pathological examination. For PrP^{Sc} purification, the whole brain hemisphere is homogenized to 10% (w/v) in a 5% glucose solution. Briefly, proteinase K (PK) is used at 10 µg/ml and digestion is blocked with PMSF. After addition of sarkosyl to 10% and Tris.HCL pH 7.4 to 10 mM, samples are incubated for 15 minutes at room temperature. They are then centrifuged at 245000 g for 4 hours at 20°C on a 10% sucrose cushion (Beckmann TL100 ultracentrifuge). Pellets are resuspended in Laemmli buffer and analyzed by Western blotting employing a polyclonal anti-mouse PrP antibody. The innoculation of the LRP^{-/-} mice with PrP^{Sc} shows that the mice are resistant towards PrP^{Sc} infection.

### Example 9: Inactivation of the LRP genes in neuroblastoma cells (N₂a) by Antisense RNA technique

### (a) Introduction

LRP expression can be downregulated in the presence of an antisense LRP mRNA. An alternative ribozyme approach is also conceivable.
First, the downregulation of LRP can be meassured in cell culture systems employing scrapie infected versus uninfected neuroblastoma cells (N₂a). Scrapie infected N₂a cells should lose their infectivity in the presence of the antisense/ribozyme construct and neuroblastoma cells should not be infectable with PrP^{Sc} any more in the presence of antisense LRP mRNA/ribozymal RNA.
Second, the same transfection vector can be microinjected into oocytes (fertilized eggs). After microinjection, survived eggs can be transferred into the oviduct of a pseudopregnant foster mother. The offsprings should show decreased levels of LRP or should fail to express LRP any more resulting in a complete resistance towards a scrapie infection.
Two examples for an antisense and an ribozyme derived approach are given below. Microinjection of in vitro transcribed RNA and antisense oligonucleotides in mouse oocytes and early embryos have been performed to study the gain and loss-of-function of genes (Kola and Sumarsono, 1996). An adenoviral VAI small RNA has been used as a carrier for cytoplasmic delivery of ribozymes(Prislei et al., 1997).

### (b) Methodology

### Construction of antisense LRP expression vector pCl-neo-asLRP

A construct spanning the cDNA from postion -65 to +901 of the mouse 37 kDa LRP ORF was generated by RT-PCR from total RNA isolated from N2a neuroblastoma cells using two oligonucleotide primers. The resulting 970 bp DNA fragment was subcloned in antisense orientation into the pCl-neo mammalian expression vector (Promega) using *Nhel* (5') and Smal (3') restriction sites. The correct insert was verified by restriction analysis and dideoxy sequencing.

### Cell Culture and Transfection

Scrapie-infected (ScN₂a) and non-infected mouse neuroblastoma cells (N₂a) were maintained in Dulbecco's modified Eagle's medium (DMEM) (Sigma) supplemented with 10 % FCS, 1 % Glutamine, 100 µg/ml penicillin and 100 µg/ml streptomycin. Cells were grown in a 25 cm² culture bottle to 70 % confluency under the above conditions and transfected in serum-free medium for 14 hours with 100 µg of Lipofectamine (Life Technologies) and 16 µg of plasmid DNA pCl-neo-asLRP. After 48 hours the medium was replenished with medium containing 400 µg/ml G418. (Life Technologies). Colonies resistant to G418 were expanded and screened for antisense LRP expression by Northern blot analysis. Furthermore LRP levels were determined by SDS-PAGE and Western-Blotting.

### Microinjection into mouse oocytes

### Restriction hydrolysis of pCl-neo-asLRP

Prior to microinjection in oocytes bacterial DNA-sequences had to be removed from the pCl-neo-asLRP plasmid. Therefor the plasmid was cleaved upstream the CMV promotor and downstream the Neomycin gene with *Bgl*II and *Bam*HI, respectively. The DNA-fragment was purified by TAE-buffered gel electrophoresis and eluted from the gel by standard procedures. After phenol extraction and ethanol precipitation the DNA was resuspended in TE-buffer (5 mM TrisHCl, pH 7.4, 0.2 mM EDTA, pH 7.4), filtered through a 0.2 µm filter and the DNA concentration determined. The DNA was diluted in TE-buffer to a concentration of 3 to 5 ng/ml and 20 ml aliquots were stored frozen until use.

### Superovulation and mating

Superovulation in female B6D2F₁ mice was induced by intraperitoeal injection of 8 IU (International Units) of PMSG (pregnant mare's serum gonadotropin). After 48 hours 7 IU of hCG (human chorionic gonadotropin) were injected intraperitoneally. Female mice were subsequently brought together with stud males of the same strain for mating. Animals were kept together overnight.
After 24 hours successul mating was verified by taking a plug control. After that female mice were killed and tubes preparated. Fertilized eggs in cumulus cells were washed out and were treated with hyaluronidase for removal of the cumulus cells. Fertilized eggs were kept in culture medium.

### Microinjection of DNA in oocytes

The isolated restriction fragment (as described above) was injected into the pronuclei of the fertilized mouse eggs using a micromanipulator. 3-5 ng of DNA were injected into 90-240 fertilized eggs. After microinjection survived eggs were transferred into the oviduct of a pseudopregnant foster mother.

Offsprings (the presence of the antisense/ribozyme cassette will be analyzed by PCR) should express antisense LRP RNA and should show decreased levels of LRP or should fail to express LRP completely. These animals should be partially or totally resistant towards a Scrapie infection.

### Example 10: Localization of LRP and PrP on the surface of neuroblastoma cells

Scrapie infected and non-infected N₂a mouse neuroblastoma cells and human osteosarcoma (HOS) cells were maintained as monolayer cultures in DMEM medium (Gibco) supplemented with 10 % FCS, 1 % Glutamine, 100 µg/ml penicillin and 100 µg/ml streptomycin. Cells were seeded half confluently on 10 mm diameter coverslips and were grown overnight for attachment. Cells were fixed with 4 % paraformaldehyd in phosphate-buffered saline (PBS) for 20 minutes at 4 °C. Fixed cells were washed three times in PBS. For cytoplasmic staining cells were permeabilized with 0.1 % Triton X-100 in PBS for 10 min at 4 °C. After rinsing three times with PBS coverslips were blocked with 10 % FCS in PBS for one hour at room temperature and washed once in PBS. For detection of LRP cells were incubated for one hour at room temperature with a monoclonal LRP-specific antibody, diluted 1:200 in 10 % FCS in PBS. Controls were incubated with pre-immuneserum in 10 % FCS in PBS. Afterwards cells were rinsed three times in PBS and a fluorescein isothiocyanate (FITC)-conjugated anti-mouse secondary antibody (Jackson ImmunoResearch Lab.) diluted 1:100 in 10 % FCS in PBS was applied for 45 minutes at room temperature. Coverslips were then washed three times with PBS and mounted in Mounting Medium (Sigma). Cells have been visualized in a immunfluorescence microscope. Magnification was from 400 fold to 630 fold.

Cultivated Murine Neuroblastoma cells (N₂a, Figure 18) and human osteosarcoma cells (HOS) have been incubated with a LRP specific antibody, followed by the use of a second FITC conjugated anti-mouse secondary antibody. The dense circle on the surface of the cells indicate that LRP is on the surface of neuronal cells. PrP^{c} appears also on the surface of the neuronal (or other Scrapie infectable) cell anchored by glycosylphosphatidyl inositol (GPI) (Rogers et al., 1991). Although treatment of the cells with phospholipase c (PIPL-c) removes PrP^{c} from the cell surface by cleaving off its GPI anchor (Caughey and Raymond, 1991), some researchers have evidence that a transmembrane region (TM) of PrP^{c} exists (Harris and Lehmann, 1996) which is responsible for fixing a mutant PrP on the cell surface. Thus, LRP and PrP are both localized on the surface of Scrapie infectable cells.

### Example 11: Uptake of recombinant PrP from human and cattle by murine neuroblastoma cells (N₂a)

For incubation experiments with recombinant prion proteins cells were incubated overnight with 3 µ g of recombinant GST::huPrP, GST::bovPrP and GST per ml of culture medium. For inhibition studies cells were pre-incubated for two hours with a LRP-specific antibody diluted 1:100 in culture medium. After incubation overnight cells were fixed, permeabilized and immunostained as described above. For detection of recombinant proteins a polyclonal anti-GST (Santa Cruz Biotechnologies) or anti-PrP 3B5 primary antibody (1:100 dilution in 10 % FCS in PBS) was used. As a secondary antibody either a Texas Red or FITC-conjugated anti-rabbit antibody (Jackson ImmunoResearch) was used.

Figure 19 (left panel) demonstrates the uptake of bovine PrP fused to GST by N₂a cells. The small dots represent the bovine PrP on the surface of N₂a cells detected by a PrP specific antibody (3B5). The uptake was prohibited when the cells have been preincubated with a LRP specific antibody (Rieger et al., 1997) (Figure 19, right panel) demonstrating that LRP is responsible for the uptake of the prion protein by N₂a cells. Thus, the LRP specific antibody can be used as a therapeutic tool to prevent the uptake of the prion protein PrP by the LRP.

The same result has been obtained when the human prion protein was used. GST alone failed to bind to the cells demonstrating that the PrP portion of the GST::PrP fusion protein is responsible for the uptake by N₂a cells.

### LIST OF REFERENCES

Albelda, S.M. and Buck, C.A. (1990) Integrins and other cell adhesion molecules. *FASEB J.* **4,** 2868-2880.

Alper, T., W.A. Cramp, D.A. Haig and M.C. Clarke. 1967. Does the agent of scrapie replicate without nucleic acid? *Nature* **214:** 764-766.

Beck K., Hunter I., Engel J. (1990) Structure and function of laminin: anatomy of a multidomain glycoprotein. *FASEB J.* **4,** 148-160.

Bignon, C., Roux-Dosseto, M., Zeigler, M.E., Mattei, M.G., Lissitzky, J.C., Wicha, M.S., and Martin, P., (1991). Genomic analysis of the 67 kDa laminin receptor in normal and pathological tissues: circumstantial evidence for retroposon features. *Genomics* **10,** 481-485

Borchelt, D.R. and Sisodia, S.S. (1996) Loss of funczional prion protein: a role in prion disorders? *Chemistry & Biology* **3,** 619-621.

Brandner S., Isenmann S., Raeber A., Fischer M., Sailer A., Kobayashi Y., Marino S., Weissmann C., Aguzzi A. (1996a) Normal host prion protein necessary for scrapie-induced neurotoxicity. *Nature* **379**:339-343.

Brandner, S., Raeber, A., Sailer, A., Blättler, T., Fischer, M., Weissmann, C. and Aguzzi, A. (1996)b) Normal host prion protein (PrP^{c}) is required for scrapie spread within the central nervous system. *Proc*. *Natl. Acad*. *Sci.* **93**, 13148-13151.

Buehler, H., Fischer, M., Lang, Y., Bluethmann, H., Lipp, H.P., DeArmond, S.J., Prusiner, S.B., Aguet, M. and Weissmann, C. Normal development and behaviour of mice lacking the neuronal cell-surface PrP protein. *Nature* **356** (1992) 577-582.

Buehler, H., A. Aguzzi, A. Sailer, R.-A. Greiner, P. Autenried, M. Aguet, and C. Weissmann. (1993) Mice devoid of PrP are resistent to scrapie. *Cell* **73**: 1339-1347.

Burgeson, R.E., Chiquet, M., Deutzmann, R., Ekblom, P., Engel, J., Kleinmann, H., Martin, G.R., Meneguzzi, G., Paulsson, M., Sanes, J., Timpl, R., Tryggvason, K., Yamada, Y. and Yurchenco, P.D. (1994) A new nomenclature for the laminins. *Matrix Biol.* **14,** 209-211.

Castronovo, V., Taraboletti, G., Sobel, M.E. (1991a) Functional domains of the 67 kDa laminin receptor precursor. *J. Biol*. *Chem.* **266,** 20440-20446.

Castronovo, V, Claysmith, A.P., Barker, K.T., Cioce V, Krutzsch, H.C., Sobel, M.E. (1991b) Biosynthesis of the 67 kDa high affinity laminin receptor precursor. *Biochem. Biophys. Res*. *Commun.* **177,** 177-183.

Caughey, B., Raymond, G.J., Ernst, D. and Race, R.E. (1991) N-terminal truncation of the Scrapie-associated form of PrP by lysosomal protease(s): Implications regarding the site of conversion of PrP to the protease-resistant state. *J. Virol.,* **65**, 6597-6603.

Caughey, B., Race, R. and Chesebro, B. Effects of scrapie infection on cellular PrP metabolism. *in* Prion Diseases of Humans and Animals. Prusiner, S.B., Collinge, J., Powell, J. and Anderton, B. Ellis Horwood limited, Chichester, England, 1992.

Collinge, J., Wittington, M.A., Sidle, K.C.L., Smith, C., J., Palmer, M.S., Clarke, A.R. and Jefferys J.G.R. (1996) Prion protein is necessary for normal synaptic function. *Nature* **370** (1994) 295-297.

Chiorini, J.A., Wendtner, C.M., Urcelay, E., Safar, B., Hallek, M. and Kotin, R.M. High-efficiency transfer of the T cell Co-stimulatory molecule B7-2 to lymphoid cells using high-titer recombinant adeno-associated virus vectors. *Hum. Gene Therapy* **6**, 1531-1541 (1995).

Danhauser-Riedl, S., Warmuth, M., Druker, B.J., Emmerich, B. and Hallek, M. Activation of Src kinases p53/561yn and p59hck by p210bcr/abl in myeloid cells. *Cancer Res.* **56,** 3589-3596.

Demianova, M., Formosa, T.G. and Ellis, S.R. (1996) Yeast proteins related to the p40/laminin receptor precursor precursor are essential components of the 40 S ribosomal subunit. *J. Biol*. *Chem.* **271,** 11383-11391.

Douville, P.J., Harvey, W.I., and Carbonette, S. (1988) Isolation and partial characterization of high affinity laminin receptor precursor in neural cells. *J. Biol. Chem.* **263,** 14964-14969.

Douville, P.J., and Carbonetto, S. (1992). Genetic linkage analysis in recombinant inbred mice of P40, a putative clone for the high affinity laminin receptor. Mamm. Genome **3**, 438-446.

Edenhofer, F., Rieger, R., Famulok, M., Wendler,W., Weiss, S. and Winnacker, E.-L. (1996) Prion protein PrP^{c} interacts with molecular chaperones of the Hsp60 familiy. *J. Virol*., **70,** 4724-4728.

Fernandez, M.-T., Castronovo, V., Rao, C.N., and Sobel, M.E. (1991). The high affinity murine laminin receptor is a member of a multicopy gene family. Biochem. Biophys. Res. Commun. **175,** 84-90.

Förster, R., Mattis, A.E., Kremmer, E., Wolf, E., Brem, G., and Lipp, M. A putative chemokine receptor, BLR1 directs B cell migration to defined lymphoid organs and specific anatomic compartments of the spleen (1996) *Cell* **87**, 1037-1047.

Garcia-Hernandez, M., Davies, E. and Staswick, P.E. (1994). Arabidopsis p40 homologue. A novel acidic protein associated with the 40S subunit of ribosomes. *J. Biol*. *Chem.* **269,** 20744-20749.

Gossen, M., and Bujard, H. (1992). Tight control of gene expression in mammalian cells by tetracycline-responsive promoters. *Proc. Natl. Acad*. *Sci.* USA **89,** 5547-5551.

Griffith, J.S. (1967) Self-replication and Scrapie. *Nature,* **215,** 1043-1044.

Gyuris, J., E. Golemis, H. Chertkov and R. Brent (1993) Cdi1, a Human G1 and S Phase Protein Phosphatase That Associates with Cdk2. Cell **75:** 791-803.

Harris, D.A. and Lehmann, S. A cell culture model of familial prion diseases. *in* Transmissible subacute spongiform encephalopathies: Prion diseases. L. Court, B. Doolet (eds.). 1996, Elsevier, Paris, 331-338.

Heukeshoven, J., and Dernick, R. Improved silver staining procedure for fast staining in phast system development unit I, Staining of sodium dodecylsulfate gels (1988) *Electrophoresis* **9,** 28-32.

Hinek A., Wrenn, D.S., Mecham, R.P. and Barondes, S.H. (1988) The elastin receptor is a galactoside binding protein. *Science* **239**, 1539-1541.

Hunter, D. D., Shah, V., Merlie, J.P. and Sanes, J.R. (1989) A laminin-like adhesive protein concentrated in the synaptic cleft of the neuromuscular junction. *Nature* **338,** 229-234.

Keppel, E., and Schaller, H.C. (1991) A 33 kDa protein with sequence homology to the laminin binding protein is associated with the cytoskeleton in hydric and mammalian cells. *J. Cell. Sci.* **100,** 789-797.

King, L.A. and R.D. Possee. 1992. *In* The Baculo expression system: A laboratory guide-1992. Chapman and Hall, London.

Klein, M.A., Frigg, R., Flechsig, E., Raeber, A.J., Kalinke, U., Bluethmann, H., Bootz, F., Suter,M., Zinkernagel, R.M. and Aguzzi, A. (1997) A crucial role for B cells in neuroinvasive scrapie. *Nature* **390,** 687-690.

Kleinmann, H.K., Ogle, R.L., Cannon, F.B., Little, C.D., Sweeny, T.M. and Luckenbill-Ebbs, L. (1988) Laminin receptor precursor for neurite formation. *Proc. Natl*., *Acad*. *Sci.* **85**, 1282-1286.

Kola, I. and Sumarsono, S.H.: Microinjection of in vitro transcribed RNA and antisense oligonucleotides in mouse oocytes and early embryos to study the gain-and loss-of-function of genes. Mol. Biotechnol. 6 (1996) 191-9.

Landowski, T.H., Dratz, E.A. and Starkey, J.R. (1995) Studies of the structure of the metastasis-associated 67 kDa laminin binding protein: Fatty acid acylation and evidence supporting dimerization of the 32 kDa gene product to form the mature protein. *Biochemistry* **34,** 11276-11287.

Lansbury, P.T.Jr. and B. Caughey. (1995) The chemistry of scrapie infection: implications of the 'ice 9' metaphor. *Chemistry & Biology* **2:** 1-5.

Leanna, C.A. and Hannick, M. The reverse two-hybrid system: a genetic scheme for selection against specific protein/protein interactions. 1996. Nucleic Acids Res. 24, 3341-3347.

Lehmann, S. and Harris, D.A. Mutant and infectious prior proteins display common biochemical properties in cultured cells. (1996) *J. Biol. Chem*. 271, 1633-1637.

Lasmézas, C.I., Deslys, J.P., Demaimay, R., Adjou, K.T., Hauw, J.J., Dormont, D., Strain specific and common pathogenic events in murine models of scrapie and bovine spongiform encephalopathy. (1996) *J. Gen*. *Virol.* **77:** 1601-1609.

Lasmézas, C.I., J.-P. Deslys, O. Robain, A. Jaegly, V. Beringue, J.-M. Peyrin, J.-G. Fournier, J.-J. Hauw, J. Rossier and D. Dormont. (1997) Transmission of the BSE agent to mice in the absence of detectable abnormal prion protein. *Science* **275:** 402-405.

Lesot, H., Kühl, U. and von der Mark, K. (1983) Isolation of a laminin binding protein from muscle cell membranes. *EMBO J.* **2,** 861-865.

Lledo P-M, Tremblay P, DeArmond SJ, Prusiner SB, Nicoll RA (1996) Mice deficient for prion protein exhibit normal neuronal excitability and synaptic transmission in the hippocampus. *Proc Natl Acad Sci USA* **93**:2403-2407.

Ludwig, G.V., Kondig, J.P. and Smith, J.F. (1996) A putative receptor for Venezuelan Equine Encephalitis virus from mosquito cells. *J. Virol.,* **70,** 5592-5599.

Malinoff, H.L. and Wicha, M.S. (1983) Isolation of a cell surface receptor for laminin from murine fibrosarcoma cells. *Biochem*. *Biophys*. *Res*. *Commun*. **111,** 804-808.

Mecham, R.P. (1991) Receptors for laminin on mammalian cells. *FASEB J.* **5,** 2538-2546.

Meyer, R.K., M.P. McKinley, K.A. Bowman, M.B. Braunfeld, R.A. Barry, and S.B. Prusiner. 1986. Separation and properties of cellular and scrapie prion proteins. *Proc. Natl*. *Acad*. *Sci.* USA **83:** 2310-2314.

Mohane, Rao, J.K, and Argos, P. (1986) A conformational preferred parameter to predict helices in integral membrane proteins. *Biochem*. *Biophys*. *Acta*, **869,** 197-214.

O'Reilly, D.R., L.K. Miller and V.A. Luckow. (1992) *In* Baculovirus expression vectors: a laboratory manual-1992. 1st ed. W.H. Freeman and Co., New York.

Ouzounis, C., Kyrpides, N. and Sander, C. (1995) Novel protein families in archaean genomes. *Nucl. Acids Res.* **23,** 565-570.

Pan, K.-M., M. Baldwin, J. Nguyen, M. Gasset, A. Serban, D. Groth, I. Mehlhorn, Z. Huang, R.J. Fletterick, F.E. Cohen and S.B. Prusiner. (1993) Conversion of α-helices into β-sheets features in the formation of the scrapie prion proteins. *Proc*. *Natl. Acad. Sci.* USA **90:** 10962-10966.

Perini, F., Vidal, R., Ghetti, B., Tagliavini, F., Frangione, B. and Prelli, F. (1996) PrP27-30 is a normal soluble prion protein fragment released by human platelets. *Biochem. Biophys. Res. Commun*., **223,** 572-577.

Prislei, S., Buonomo, S.B., Michienzi, A. and Bozzoni, I.: Use of adenoviral VAI small RNA as a carrier for cytoplasmic delivery of ribozymes. RNA 3 (1997) 677-87.

Prusiner, S.B., McKinley, M.P., Groth, D.F., Bowman, K.A., Mack, N.l., Cochran, S.P., and Masiarz, F.R. (1981) Scrapie agent contains a hydrophobic protein. *Proc. Natl*. *Acad*. *Sci*. *U.S.A.,* **78,** 6675-6679.

Prusiner, S.B. (1982) Novel proteinaceous infectious particles cause Scrapie. *Science,* **216,** 136-144.

Prusiner, S.B., McKinley, M.P, Bowman, K.A., Bolton, D.C, Bendheim, P.E., Groth, D.F., and Glenner, G.G. (1983) Scrapie prions aggregate to form amyloid-like birefringent rods. *Cell,* **35,** 349-358.

Prusiner, S.B., Groth, D.F., Bolton, D.C., Kent, S.B. and Hood, L.E. (1984) Purification and structural studies of a major scrapie prion protein. *Cell,* **38**, 127-134.

Prusiner, S.B. (1991) Molecular biology of prion disease. *Science* **252,** 151 5-1522.

Rao, N.C., Barsky, S.H., Terranova, V.P. and Liotta, L.A. (1983) Isolation of a tumor cell laminin receptor precursor. *Biochem*. *Biophys*. *Res*. *Commun.* **111,** 804-808.

Rao, C.N., Castronovo, V., Schmitt, M.C., Wewer, U.M., Claysmith, A.P., Liotta, L.A. and Sobel, M.E. (1989) Evidence for a precursor of the high-affinity metastasis-associated murine laminin receptor precursor. *Biochemistry* **28,** 7476-7486.

Rieger, R., Edenhofer, F., Lasmézas, C.I. and Weiss, S.: The human 37-kDa laminin receptor precursor interacts with the prion protein in eukaryotic cells. (1997) *Nat. Med*. **3,** 1383-1388.

Sambrook, J., Fritsch, E. F. and Maniatis, T. (1989) Molecular cloning: a laboratory manual, 2nd ed., Cold Spring Harbor Laboratory Press, Cold Spring Habor, NY.

Shyng, S.L., Heuser, J.E. and Haris, D.A.: A glycolipid-anchored prion protein is endocytosed via clathrin-coated pits. (1994) *J. Cell. Biol*. **125,**1239-1250.

Shyng, S.-L., Moulder, K.L., Lesko, A., Harris, D.A. The N-terminal domain of a glycolipid-anchored prion protein is essential for its endocytosis via clathrin-coated pits. (1995) *J. Biol*. *Chem.* **270,** 14793-14800:

Stahl N., Borchelt D.R., Hsiao K., Prusiner, S.B. Scrapie prior proteins contains a phosphatidylinositol glycopipid. (1987) *Cell* **51,** 229-240.

Stahl, N., M.A. Baldwin, D.B. Teplow, L. Hood, B.W. Gibson, A.L. Burlingame and S.B. Prusiner. (1993) Structural studies of the scrapie prion protein using mass spectrometry and amino acid sequencing. *Biochemistry* **32**: 1991-2002.

Tandon, N.N., Holland, E.A., Kralisz, U., Kleinmann, H.K., Robey, F.A. and Jamieson, G.A. (1991) Interaction of human platelets with laminin and identification of the 67 kDa laminin receptor precursor on platelets. *Biochem. J*. **274,** 535-542 Taraboulos A., Borchelt, D.R., McKinley, M.P., Raeber, A., Serban, D., DeArmond, S.J. and Prusiner, S.B. Dissecting the pathway of scrapie prior synthesis in cultured cells. *in* Prion diseases of humans and Animals. Prusiner, S.B., Collinge, J., Powell, J. and Anderton, B. Ellis Horwood limited, Chichester, England, 1992.

Taraboulos A., Scott, M., Sernenov, A., Avraham, D., Laszlo, L., Prusiner, S.B. (1995) Cholesterol depletion and modification of COOH-terminal targetting sequence of the prior protein inhibit formation of the scrapie isoform. *J. Cell. Biol.* **129,** 121-132.

Vey, M., Pilkuhn, S., Wille, H., Nixon, R., DeArmond, S.J., Smart, E.J., Anderson, R.G., Rtaraboulos, A., and Prusiner, S.B. Subcellular colocalization of the cellular and scrapie prion proteins in caveolae-like membranous domains. (1996) *Proc. Natl. Acad. Sci*. *U.S.A.* **93,** 14945-14949.

Wang, K.-S., Kuhn, R.J., Strauss, E.G., Ou, S. and Strauss, J.H. (1992) High affinity laminin receptor precursor is a receptor for Sindbis Virus in mammalian cells. *J. Virol.* **66,** 4992-5001.

Wang, Y.-H., A.H. Davies, and I.M. Jones. (1995) Expression and purification of glutathione S-transferase tagged HIV-1 gp120.*Virology*, **208**: 142-146,

Weiss, S., Famulok, M., Edenhofer, F., Wang, Y.-H., Jones, I.M., Groschup, M. and Winnacker, E.-L. (1995) Overexpression of Active Syrian Golden Hamster Prion Protein PrP^{c} as a Glutathione S-Transferase Fusion in Heterologous Systems. *J. Virol*., **69,** 4776-4783.

Weiss, S., Rieger, R., Edenhofer, F., Fisch, E. and Winnacker, E.-L. (1996) Recombinant prion protein rPrP27-30 from Syrian Golden hamster reveals proteinase K sensitivity. *Biochem*. *Biophys*. *Res*. *Commun.,* **219,** 173-179.

Weissmann, C. (1994) Molecular biology of prion diseases. *Trends Cell. Biol.* **4:** 10-14.

Wendler, W., H. Altmann and E.-L. Winnacker. (1994) Transcriptional activation of NFI/CTF1 depends on a sequence motif strongly related to the carboxyterminal domain of RNA polymerase II. *Nucl*. *Acids Res.* **22:** 2601-2603.

Wewer, U.M., Liotta, L., Jaye, M., Ricca, G.A., Drohan, W.N., Claysmith, A., P., Rao, C.N., Wirth, P., Coligan, J.E., Albrechtsen, R., Mudry,M. and Sobel, M.E. (1986) *Proc*. *Natl*. *Acad*. *Sci. U*.*S*.*A*.**83,** 7137-7141.

Whitford, M., S. Stewart, J. Kuzio and P. Faulkner. (1989) Identification and sequence analysis of a gene encoding gp67, an abundant envelope glycoprotein of the baculovirus *autographica californica* nuclear polyhedrosis virus. *J. Virol.* **63:** 1393-1399 .

Yow, H., Wong, J.M., Chen, H.S., Lee, C., Steele, G.D.J. and Chen, L.B. (1988) Increased mRNA expression of a laminin-binding protein in human colon carcinoma: complete sequence of a full-length cDNA encoding the protein. *Proc*. *Natl*. *Acad*. *Sci.* **85,** 6394-6398.

## Claims

1. Method of diagnosis of transmissible spongiform encephalopathy (TSE), said method comprising the following step:
determining the over-expression of a marker protein for TSE in a sample to be tested.

2. Method of diagnosis of transmissible spongiform encephalopathy (TSE), said method comprising the steps of:
(a) determining the expression of a marker protein for TSE in a sample to be tested; and
(b) comparing the value obtained with a control value, wherein an increase in the expression level is indicative of the occurence of TSE.

3. Method accoding to anyone of claims 1 or 2, wherein the sample is a homogenate from the brain, the spleen, the pancreas, the liver or the muscle.

4. Method according to anyone of claims 1 to 3, wherein the marker protein is the laminin receptor (LR) or the laminin receptor precursor (LRP).

5. Method according to anyone of claims 1 to 4, wherein the marker protein is the human laminin receptor (LR) or the human laminin receptor precursor (LRP).

6. Method according to anyone of claims 1 to 4, wherein the marker protein is the bovine laminin receptor (LR) or the bovine laminin receptor precursor (LRP).

7. Method according to anyone of claims 1 to 4, wherein the marker protein is the ovine laminin receptor (LR) or the ovine laminin receptor precursor (LRP).

8. Method according to anyone of claims 1 to 4, wherein the marker protein is the feline laminin receptor (LR) or the feline laminin receptor precursor (LRP).

9. Method according to anyone of claims 1 to 8, wherein the marker protein is determined by a "standard" immune test.

10. Method according to claim 9, wherein said "standard" immune test is a Western Blot test.

11. Method according to anyone of claims 1 to 8, wherein the sample is incubated with antibodies which are specific to the laminin receptor (LR) or the laminin receptor precursor (LRP).

12. Method according to anyone of claims 1 to 8, wherein the altered expression of the laminin receptor or the laminin receptor precursor as the marker for TSE is determined by standard molecular biology methods.

13. Method according to anyone of claims 1 to 12, wherein said sample is derived from an organism selected from the group consisting of a cow, a human, a sheep or a feline animal.

14. Diagnostic test kit for detecting a transmissible spongiform encephalopathy (TSE), **characterized in that** it contains antibodies specific to the laminin receptor precursor (LRP) and/or the laminin receptor (LR) for detecting the altered expression of the laminin receptor (LR) and/or the laminin receptor precursor (LRP) as a marker protein for TSE by a method according to anyone of claims 1 to 13.

15. Diagnostic test kit according to claim 14, wherein said antibodies are antibodies against bovine, human, ovine and/or feline LRP/LR.

16. Diagnostic test kit according to anyone of claims 14 or 15, **characterized in that** the antibodies are monoclonal.

17. Diagnostic test kit according to anyone of claims 14 or 15, **characterized in that** the antibodies are polyclonal.

18. Diagnostic test kit according to anyone of claims 14 to 17, containing recombinant laminin receptor precursor fused to GST (GST::LRP) synthesized in the baculovirus system as a positive control and recombinant GST synthesized in the baculovirus system as a negative control in the test.

19. Diagnostic test kit for detecting a transmissible spongiform encephalopathy (TSE), **characterized in that** it contains a nucleic acid which is capable of hybridising under stringent conditions with the nucleic acid coding for the laminin receptor precursor (LRP) and/or the laminin receptor (LR), as well as the other necessary elements for performing a method according to claims 14 to 18.

20. Use of an antibody capable of binding the laminin receptor (LR) and/or the laminin receptor precursor (LRP) for the preparation of a composition for diagnosing a transmissible spongiform encephalopathy (TSE).

21. Use of a nucleic acid capable of hybridizing under stringent conditions with the nucleic acid coding for the laminin receptor precursor (LRP) and/or the laminin receptor (LR) for the preparation of a composition for diagnosing a transmissible spongiform encephalopathy (TSE).

22. Use according to anyone of claims 20 or 21, wherein the occurence of TSE is indicated by over-expression of the laminin receptor precursor (LRP) and/or the laminin receptor (LR) .
